# EUROPÄISCHE PATENTSCHRIFT

(11) **EP 1 472 357 B1**
(45) Veröffentlichungstag und Bekanntmachung des Hinweises auf die Patenterteilung: **16.12.2009**
(21) Anmeldenummer: 03704382.5
(22) Anmeldetag: 13.01.2003
(51) Int. Cl.: C12N 15/82

(54) **VERFAHREN ZUR HERSTELLUNG MEHRFACH UNGESÄTTIGTER FETTSÄUREN MITTELS EINES NEUEN ELONGASE-GENS**
METHOD FOR PRODUCING POLYUNSATURATED FATTY ACIDS BY MEANS OF A NEW ELONGASE-GENE
PROCEDE DE PRODUCTION D'ACIDES GRAS POLY-INSATURES UTILISANT UN NOUVEAU GENE D'ELONGASE

(30) Priorität: 30.01.2002 DE 10203713; 11.02.2002 DE 10205607
(43) Veröffentlichungstag der Anmeldung: 03.11.2004
(73) Patentinhaber: BASF Plant Science GmbH, 67056 Ludwigshafen (DE)
(72) Erfinder: LERCHL, Jens, S-268 31 Svalöv (SE); HEINZ, Ernst, 22609 Hamburg (DE); ZANK, Thorsten, 22303 Hamburg (DE)
(86) Internationale Anmeldenummer: PCT/EP2003/000221
(87) Internationale Veröffentlichungsnummer: WO 2003/064638

(56) Entgegenhaltungen:
- WO-A-98/46765
- WO-A-99/64616
- DATABASE EMBL [Online] Phytophthora infestans EST, 24. Mai 2003 (2003-05-24) retrieved from EBI Database accession no. cd257414 XP002247892 & KAMOUN,S. ET AL.: "Initial assessment of gene diversity for the oomycete pathogen Phytophthora infestans based on expressed sequences" FUNGAL GENET BIOL, Bd. 28, Nr. 2, 1999, Seiten 94-106,
- DATABASE EMBL [Online] Phytophthora infestans EST, 21. September 2001 (2001-09-21) retrieved from EBI Database accession no. be775687 XP002247893 & KAMOUN S. ET AL.: "Initial assessment of gene diversity for the oomycete pathogen Phytophthora infestans based on expressed sequences" FUNGAL GENET BIO., Bd. 28, Nr. 2, 1999, Seite 94-106
- CERTIK M ET AL: "Desaturase-defective fungal mutants: useful tools for the regulation and overproduction of polyunsaturated fatty acids" TRENDS IN BIOTECHNOLOGY, ELSEVIER PUBLICATIONS, CAMBRIDGE, GB, Bd. 16, Nr. 12, 1. Dezember 1998 (1998-12-01), Seiten 500-505, XP004143810 ISSN: 0167-7799
- ABBADI A ET AL: "Transgenic oilseeds as sustainable source of nutritionally relevant C20 and C22 polyunsaturated fatty acids?" EUROPEAN JOURNAL OF LIPID SCIENCE AND TECHNOLOGY, WILEY VCH VERLAG, WEINHEIM, DE, Bd. 103, Nr. 2, Februar 2001 (2001-02), Seiten 106-113, XP002228744 ISSN: 1438-7697

## Beschreibung

Diese Erfindung betrifft Verfahren zur Herstellung von C20 oder C22 Fettsäuremolekülen mit mindestens zwei Doppelbindungen wobei ein Elongasegen mit der Sequenz SEQ ID NO:1 oder seine Homologa, Derivate oder Analoga, ein Genkonstrukt, das dieses Gen oder seine Homologa, Derivate oder Analoga umfasst, eingesetzt wird.

Bestimmte Produkte und Nebenprodukte natürlich vorkommender Stoffwechselprozesse in mikrobiellen Zellen oder in den Zellen von Tieren und vorteilhaft Pflanzen sind für ein breites Spektrum an Industrien, einschließlich der Futtermittel-, Nahrungsmittel-, Kosmetik- und pharmazeutischen Industrie, nützlich. Zu diesen gemeinsam als "Feinchemikalien" bezeichneten Molekülen gehören auch Lipide und Fettsäuren, unter denen eine beispielhafte Klasse die mehrfach ungesättigten Fettsäuren sind. Mehrfach ungesättigte Fettsäuren (polyunsaturated fatty acids, PUFAs) werden beispielsweise Nahrungsmittel für Kinder zugegeben, um einen höheren Nährwert dieser Nahrungsmittel zu erzeugen. PUFAs haben zum Beispiel einen positiven Einfluss auf den Cholesterinspiegel im Blut von Menschen und eignen sich daher zum Schutz gegen Herzkrankheiten. Feinchemikalien wie mehrfach ungesättigte Fettsäuren (polyunsaturated fatty acids, PUFAs) lassen sich aus tierischen Quellen, wie beispielsweise Fisch, isolieren oder mit Mikroorganismen durch Züchtung von Mikroorganismen, die so entwickelt worden sind, dass sie große Mengen eines oder mehrerer gewünschter Moleküle produzieren und akkumulieren oder sezernieren, im großen Maßstab herstellen.

Besonders geeignete Mikroorganismen zur Herstellung von PUFAs sind Mikroorganismen, wie die Algen Phaeodactylum tricornutum oder Crypthecodinium-Arten, Ciliaten, wie Stylonichia oder Colpidium, Pilze, wie Mortierella, Entomophthora, Mucor. Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Selektion von Stämmen mit verbesserter Produktion eines bestimmten Moleküls ist jedoch ein zeitraubendes und schwieriges Verfahren.

Alternativ kann die Produktion von Feinchemikalien geeigneterweise über die Produktion von Pflanzen, die so entwickelt sind, dass sie die vorstehend genannten PUFAs herstellen, im großen Maßstab durchgeführt werden. Besonders gut für diesen Zweck geeignete Pflanzen sind Ölfruchtpflanzen, die große Mengen an Lipidverbindungen enthalten, wie Raps, Canola, Lein, Soja, Sonnenblumen, Disteln, Borretsch und Nachtkerze. Aber auch andere Nutzpflanzen, die Öle oder Lipide und Fettsäuren enthalten, sind gut geeignet, wie in der eingehenden Beschreibung dieser Erfindung erwähnt. Mittels herkömmlicher Züchtung, über die Selektion geeigneter Pflanzen, ist eine Reihe von Mutantenpflanzen entwickelt worden, die ein Spektrum an wünschenswerten Lipiden und Fettsäuren, Cofaktoren und Enzymen produzieren. Die Selektion neuer Pflanzensorten mit verbesserter Produktion eines bestimmten Moleküls ist jedoch ein zeitaufwändiges und schwieriges Verfahren oder sogar unmöglich, wenn die Verbindung in der entsprechenden Pflanze nicht natürlich vorkommt, wie im Fall von mehrfach ungesättigten C₂₀-Fettsäuren und solchen mit längeren Kohlenstoffketten.

Aufgrund der positiven Eigenschaften ungesättigter Fettsäuren hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Δ-6-Desaturasen werden in WO 93/06712, US 5,614,393, WO 96/21022 und WO 99/27111 beschrieben. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. In WO 96/13591 wird eine Δ-6-Palmitoyl-ACP-Desaturase beschrieben und beansprucht. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al., Methods in Enzymol. 71, 1981: 12141-12147, Wang et al., Plant Physiol. Biochem., 26, 1988: 777-792).

In Hefen konnte sowohl eine Verschiebung des Fettsäurespektrums zu ungesättigten Fettsäuren hin als auch eine Steigerung der Produktivität nachgewiesen werden (siehe Huang et al., Lipids 34, 1999: 649-659, Napier et al., Biochem. J., Vol. 330, 1998: 611-614). Die Expression der verschiedenen Desaturasen in transgenen Pflanzen zeigte allerdings nicht den gewünschten Erfolg. Eine Verschiebung des Fettsäurespektrum zu ungesättigten Fettsäuren hin konnte gezeigt werden, gleichzeitig zeigte sich aber, dass die Syntheseleistung der transgenen Pflanzen stark nachließ, das heißt gegenüber den Ausgangspflanzen konnten nur geringere Mengen an Ölen isoliert werden.

Die Klonierung und Expression von Elongasen, die ungesättigte Fettsäuren als Substrat der enzymatischen Reaktion um mindestens zwei C-Atome elongieren, wurde bisher weder in Hefen noch in Pflanzen beschrieben.

Das heißt weder in Hefen noch in Kulturflanzen werden natürlicherweise mehrfach ungesättigte C₂₀- und/oder C₂₂-Fettsäuren mit mindestens zwei Doppelbindungen im Fettsäuremolekül wie Arachidonsäure (ARA) und/oder Eicosapentaensäure (EPA) und/oder Docosahexaensäure (DHA) hergestellt.

Nach wie vor besteht daher ein großer Bedarf an Genen, die für Enzyme kodieren, die an der Biosynthese ungesättigter Fettsäuren beteiligt sind und es ermöglichen, diese in einem technischen Maßstab herzustellen. Ein besonders hoher Bedarf besteht für Elongasen, die ungesättigte Fettsäuren um mindestens zwei C-Atome elongieren. Keines der bisher bekannten biotechnologischen Verfahren zur Herstellung von mehrfach ungesättigten Fettsäuren liefert die vorgenannten Fettsäuren in wirtschaftlich nutzbaren Mengen.

Bei der Expression von Genen in Pflanzen gibt es immer wieder Probleme, dass heißt es kommt durch die Expression nicht zur erwarteten Steigerung bei der Herstellung des gewünschten Wertprodukts.

Es bestand daher die Aufgabe, Verfahren für die Synthese ungesättigter Fettsäuren wie mehrfach ungesättigte C₂₀- und/oder C₂₂-Fettsäuren mit mindestens zwei Doppelbindungen im Fettsäuremolekül wie Arachidonsäure (ARA) und/oder Eicosapentaensäure (EPA) und/oder Docosahexaensäure (DHA) zur Verfügung zu stellen.

Diese Aufgabe wurde durch das Verfahren nach Anspruch 1 gelöst.

Hierbei wird eine Nukleinsäure eingesetzt umfassend eine Nukleotidsequenz, die ein Polypeptid kodiert, das C₁₆- oder C₁₈-Fettsäuren mit mindestens zwei Doppelbindungen im Fettsäuremolekül verlängert, ausgewählt aus der Gruppe, bestehend aus
a) einer in SEQ ID NO:1 dargestellten Nukleinsäuresequenz,
b) einer Nukleinsäuresequenz, die sich gemäß dem degenerierten genetischen Code von der in SEQ ID NO:2 dargestellten Sequenz ableitet,
c) Derivate der in SEQ ID NO:1 dargestellten Sequenz, die Polypeptide mit mindestens 50 % Homologie zu der Sequenz kodiert, die die Aminosäuresequenzen in SEQ ID NO:2 kodiert, wobei die Sequenz als C₁₆- oder C₁₈-Elongase wirkt.

Vorteilhaft verlängert die Nukleotidsequenz ausgewählt aus der vorgenannten Gruppe von C₁₆- oder C₁₈-Fettsäuren mit mindestens zwei Doppelbindungen in der Fettsäure um mindestens zwei Kohlenstoffatome, wobei die folgenden Fettsäuren C_{18:3}^{Δ5t,9,12}, C_{20:3}^{Δ8,11,14}, C_{20:4}^{Δ5,8,11,14} und C_{20:5Δ5,8,11,14,17} jedoch nicht verlängert werden. Elongasen zeigen eine Präferenz gegenüber C_{18:3}^{Δ6,9,12}-, C_{18:4}^{Δ6,9,12,15}- und C_{16:3}^{Δ7,10,13}-Fettsäuren, die mindestens um den Faktor 1,5, bevorzugt mindestens um den Faktor 1,6, besonders bevorzugt mindestens um den Faktor 1,7 oder ganz besonders bevorzugt mindestens um den Faktor 1,8 höher ist als gegenüber den ungesättigten Fettsäuren wie C_{18:2}^{Δ9,12}-, C_{18:3}^{Δ4,7,10}-, C_{18:3} ^{Δ5,8,11}- ,C_{18:3}^{Δ7,10,13}-, C_{18:3}^{Δ8,11,14}-, C_{18:3} ^{Δ9,12,15}- oder C_{18:3} ^{Δ5,c9,12}-Fettsäuren.

Die Nukleinsäuresequenzen, die C₁₆- oder C₁₈-Fettsäuren elongieren, stammen ursprünglich vorteilhaft aus Pilzen bevorzugt aus Pilzen wie den Oomyzeten zum Beispiel Oomyzeten wie die der Gattung Phytophthora, besonders bevorzugt aus Oomyzeten der Gattung und Art Phytophthora infestans.

Die Nukleinsäuren können im erfindungsgemäßen Verfahren zur Modifikation von Ölen, Fettsäuren, Lipiden, von Lipiden stammenden Verbindungen und am stärksten bevorzugt zur Herstellung mehrfach ungesättigter Fettsäuren verwendet werden.

Als Wirtsorganismen für die Nukleinsäuren eigenen sich vorteilhaft Mikroorganismen, wie Phaeodactylum, Colpidium, Mortierella, Entomophthora, Mucor, Crypthecodinium sowie andere Algen und Pilze und Pflanzen, insbesondere Ölfruchtpflanzen, die in der Industrie zur Produktion einer Vielzahl von Feinchemikalien im großen Maßstab verwendet werden.

Mit den in beispielsweise in WO 98/01572 offenbarten Klonierungsvektoren und Techniken zur genetischen Manipulation der obengenannten Mikroorganismen und Ciliaten oder den in Falciatore et al., 1999, Marine Biotechnology 1(3):239-251, sowie Dunahay et al., 1995, Genetic transformation of diatoms, J. Phycol. 31:10004-1012 und den Zitaten darin für Algen und verwandten Organismen, wie Phaeodactylum tricornutum beschriebenen Methoden und Vektoren, können die Nukleinsäuremoleküle zur gentechnologischen Veränderung dieser Organismen verwendet werden, so dass sie bessere oder effizientere Produzenten einer oder mehrerer Feinchemikalien werden. Diese verbesserte Produktion oder Effizienz der Produktion einer Feinchemikalie kann durch eine direkte Wirkung der Manipulation einer erfindungsgemäßen Nukleinsäure vorteilhaft in Form des gesamten Gens oder durch eine indirekte Wirkung dieser Manipulation hervorgerufen werden.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Auch pilzliche Systeme wie Oomyzeten (Eukarioten/Stramenopiles/Oomyzeten/Phythialen/Pythiaceen) stellen die vorgenannten Fettsäuren her. Daher eignen sich Nukleinsäuremoleküle, die einem Oomyzet wie Phytophtohora infestans stammen, besonders zur Modifikation des Lipid- und PUFA-Produktionssystems in einem Wirt, insbesondere in Mikroorganismen, wie den vorstehend erwähnten Mikroorganismen, und Pflanzen, wie Ölfruchtpflanzen, beispielsweise Raps, Canola, Lein, Soja, Sonnenblumen, Borretsch. Ferner können Nukleinsäuren aus einem Oomyzet wie Phytophtohora infestans zur Identifikation solcher DNA-Sequenzen und Enzyme in anderen Arten, die sich zur Modifikation der Biosynthese von Vorläufermolekülen von PUFAs in den entsprechenden Organismen eignen, verwendet werden.

Der Pilz Phytophthora infestans ist ein Mitglied der Oomyzeten. Er ist mit anderen Pilzen verwandt, die in Abwesenheit von Licht wachsen kann. Pilze, wie Phytophthora, sind auf DNA-Sequenz- und Polypeptid-Ebene sehr homolog zueinander, was die Verwendung von heterologem Screening von DNA-Molekülen mit von anderen Pilzen oder Organismen stammenden Sonden ermöglicht, so dass bei vorhanden sein weiterer Nukleinsäuresequenzen neben der oben genannten Sequenz eine Konsensussequenz abgeleitet werden kann, die sich zum heterologen Screening oder zur funktionellen Kommentierung und Vorhersage von Genfunktionen in dritten Arten eignet. Derzeit ist eine Vorhersage der Funktion der durch die Sequenzen kodierten Proteine bzw. Enzyme jedoch noch nicht möglich. Die Fähigkeit, diese Funktionen zu identifizieren, z.B. die Vorhersage der Substratspezifität von Enzymen, kann daher von signifikanter Bedeutung sein. Ferner können diese Nukleinsäuremoleküle als Bezugssequenzen zur Kartierung anderer Pilze oder zur Ableitung von PCR-Primern dienen.

Im Rahmen der Erfindung wurde ein funktionell aktives PSE Gen aus dem Oomyzeten Phytophthora infestans isoliert (Eukarioten/Stramenopiles, Oomyceten/Phythialen/Pythiaceen). Das Gen ist vorteilhaft zur Produktion langkettiger mehrfach ungesättigter Fettsäuren geeignet, vorzugsweise mit mehr als sechzehn oder achtzehn Kohlenstoffatomen im Kohlenstoffgrundgerüst der Fettsäure und/oder mindestens zwei Doppelbindungen in der Kohlenstoffkette, geeignet ist, wobei die durch die Sequenz kodierten Enzyme eine Präferenz gegenüber den oben genannten Fettsäuren bei der Elongierung aufweisen.

Dieses Nukleinsäuremolekül kodiert für ein Protein, das hier als PUFA-spezifische Elongase (PSEs oder im Singular PSE bezeichnet werden). Diese PSEs können beispielsweise eine Funktion ausüben, die am Stoffwechsel (z.B. an der Biosynthese oder am Abbau) von Verbindungen, die zur Lipid- oder Fettsäuresynthese notwendig sind, wie PUFAs, beteiligt sind oder am Transmembrantransport einer oder mehrerer Lipid-/Fettsäureverbindungen entweder in die oder aus der Zelle teilnehmen.

In dieser Anmeldung wird die Funktion der Sequenz eingehender dargestellt. Wir haben ein funktionell aktives Oomycetengen isoliert, das zur Produktion langkettiger mehrfach ungesättigter Fettsäuren, vorzugsweise mit mehr als sechzehn oder achtzehn Kohlenstoffatomen im Kohlenstoffgrundgerüst der Fettsäure und/oder mindestens zwei Doppelbindungen in der Kohlenstoffkette, geeignet ist, wobei die durch die Sequenzen kodierten Enzyme eine Präferenz gegenüber den oben genannten Fettsäuren bei der Elongierung aufweisen. Daher ist hierin von einem PSE-Gen oder - Protein die Rede. Andere Veröffentlichungen und Patente offenbaren oder zeigen kein funktionell aktives PSE-Gen, obgleich es verschiedene bekannte Patentanmeldungen gibt, welche die Verlängerung gesättigter Fettsäuren mit kurzer oder mittlerer Kette (WO 98/46776 und US 5,475,099) oder die Verlängerung oder Produktion langkettiger Fettsäuren, die dann aber nicht mehr als eine Doppelbindung haben oder zu langkettigen Fettsäure-Wachsestern führen (siehe: WO 98/54954, WO 96/13582, WO 95/15387) zeigen.

WO 99/64616, WO 98/46763, WO 98/46764, WO 98/46765 beschreiben zwar die Herstellung von PUFAs in transgenen Pflanzen und zeigen die Klonierung und funktionelle Expression entsprechender Desaturase-Aktivitäten, insbesondere aus Pilzen, zeigen aber kein unumgängliches PSE-kodierendes Gen und keine funktionelle PSE-Aktivität. Die Herstellung einer Triensäure mit C₁₈-Kohlenstoffkette ist gezeigt und anhand von gamma-Linolensäure beansprucht worden, es wurde jedoch bisher nicht die Herstellung sehr langkettiger mehrfach ungesättigter Fettsäuren (mit C₂₀- und längerer Kohlenstoffkette sowie von Triensäuren und höher ungesättigten Typen) und die Substratspezifität der Elongase gelehrt.

Zur Herstellung langkettiger PUFAs müssen die mehrfach ungesättigten C₁₆- und/oder C₁₈-Fettsäuren durch die enzymatische Aktivität einer Elongase um mindestens zwei Kohlenstoffatome verlängert werden. Die Nukleinsäuresequenz kodiert die erste pilzliche Elongase, die C₁₆- und/oder C₁₈-Fettsäuren mit mindestens zwei Doppelbindungen in der Fettsäure um mindestens zwei Kohlenstoffatome verlängern kann. Nach einer Elongationsrunde führt diese Enzymaktivität zu C₂₀-Fettsäuren, und nach zwei, drei und vier Elongationsrunden zu C₂₂-, C₂₄- oder C₂₆-Fettsäuren. Von Vorteil bei der enzymatischen Aktivität ist, dass nicht alle ungesättigten C₂₀-Fettsäuren verlängert werden. Dies ermöglicht die spezifische Synthese einzelner wünschenswerter ungesättigter Fettsäuren bzw. Fettsäuregemische. Mit der oben genannten Elongase können auch längere PUFAs synthetisiert werden. Die Aktivität der Elongasen führt vorzugsweise zu C₂₀- und/oder C₂₂-Fettsäuren mit mindestens zwei Doppelbindungen im Fettsäuremolekül, vorzugsweise mit drei oder vier Doppelbindungen, besonders bevorzugt sind Fettsäuren mit einer Doppelbindung in Δ6 Position. Nachdem die Verlängerung mit den Enzymen stattgefunden hat, können weitere Desaturierungsschritte erfolgen. Daher führen die Produkte der Elongaseaktivität und der möglichen weiteren Desaturierung zu bevorzugten PUFAs mit einem höheren Desaturierungsgrad, wie Docosadiensäure, Arachidonsäure, ω6-Eicosatriendihomo-γ-linolensäure, Eicosapentensäure, ω3-Eicosatriensäure, ω3-Eicosatetraensäure, Docosapentaensäure oder Docosahexaensäure. Substrate der Enzymaktivität sind zum Beispiel Taxolsäure; 6,9-Octadecadiensäure, Linolsäure, γ-Linolensäure, Pinolensäure, α-Linolensäure oder Stearidonsäure. Bevorzugte Substrate sind Linolsäure, γ-Linolensäure und/oder α-Linolensäure. Die C₁₆- und/oder C₁₈-Fettsäuren mit mindestens zwei Doppelbindungen in der Fettsäure können durch die enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Unter der Verwendung von Klonierungsvektoren, die zur Verwendung in Pflanzen und zur Pflanzentransformation geeignet sind, wie denjenigen, die in Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)) und den in diesen Schriften genannten Zitaten veröffentlicht worden sind, lassen sich die im erfindungsgemäßen Verfahren eingesetzten Nukleinsäuren zur gentechnologischen Veränderung eines breiten Spektrums an Pflanzen verwenden, so dass diese ein besserer oder effizienterer Produzent eines oder mehrerer von Lipiden hergeleiteter Produkte, wie PUFAs, wird. Diese verbesserte Produktion oder Effizienz der Produktion eines von Lipiden hergeleiteten Produktes, wie PUFAs, kann durch direkte Wirkung der Manipulation oder eine indirekte Wirkung dieser Manipulation hervorgerufen werden.

Es gibt eine Reihe von Mechanismen, durch die die Veränderung eines PSE-Proteins die Ausbeute, Produktion und/oder Effizienz der Produktion einer Feinchemikalie aus einer Ölfruchtpflanze oder einem Mikroorganismus aufgrund eines veränderten Proteins direkt beeinflussen kann. Die Anzahl oder Aktivität des PSE-Proteins, -nukleinsäure und/oder -Gens kann erhöht sein, so dass größere Mengen dieser Verbindungen de novo hergestellt werden, weil den Organismen diese Aktivität und Fähigkeit zur Biosynthese vor dem Einbringen der entsprechenden Nukleinsäure und/oder des entsprechenden Gens fehlte.

Das Einbringen einer oben genannten Nukleinsäure und/oder eines PSE-Gens in einen Pflanze oder einen Mikroorganismus kann nicht nur den Biosynthesefluss zum Endprodukt erhöhen, sondern auch die entsprechende Triacylglycerin-Zusammensetzung erhöhen oder de novo schaffen. Ebenso kann die Anzahl oder Aktivität anderer Gene, die am Import von Nährstoffen, die zur Biosynthese einer oder mehrerer Feinchemikalien (z.B. Fettsäuren, polaren und neutralen Lipiden) nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Fettsäuren und Lipide sind selbst als Feinchemikalien wünschenswert; durch Optimierung der Aktivität oder Erhöhung der Anzahl einer oder mehrerer PSEs, die an der Biosynthese dieser Verbindungen beteiligt sind, oder durch Zerstören der Aktivität einer oder mehrerer PSEs, die am Abbau dieser Verbindungen beteiligt sind, kann es möglich sein, die Ausbeute, Produktion und/oder Effizienz der Produktion von Fettsäure- und Lipidmolekülen aus Pflanzen oder Mikroorganismen zu steigern.

Die Mutagenese des PSE-Gens bzw. der Nukleinsäure kann auch zu einem PSE-Protein mit geänderten Aktivitäten führen, welche die Produktion einer oder mehrerer gewünschter Feinchemikalien direkt oder indirekt beeinflussen. Beispielsweise kann die Anzahl oder Aktivität des PSE-Gens bzw. der Nukleinsäure gesteigert werden, so dass die normalen Stoffwechselabfälle oder - nebenprodukte der Zelle (deren Menge möglicherweise aufgrund der Überproduktion der gewünschten Feinchemikalie erhöht ist) effizient exportiert werden, bevor sie andere Moleküle oder Prozesse innerhalb der Zelle (welche die Lebensfähigkeit der Zelle senken würden) zerstören oder die Biosynthesewege der Feinchemikalie stören würden (wodurch die Ausbeute, Produktion oder Effizienz der Produktion der gewünschten Feinchemikalie verringert wird). Ferner können die relativ großen intrazellulären Mengen der gewünschten Feinchemikalie selbst toxisch für die Zelle sein oder Enzym-Rückkopplungsmechanismen, wie die allosterische Regulation, stören, beispielsweise könnte sie durch Steigerung der Aktivität oder Anzahl anderer stromabwärts folgender Enzyme oder Entgiftungsenzyme des PUFA-Wegs die Allokation der PUFA in die Triacylgylcerin-Fraktion steigern, man könnte die Lebensfähigkeit von Saatzellen erhöhen, was wiederum zu besserer Entwicklung von Zellen in Kultur oder zu Saaten führt, die die gewünschte Feinchemikalie produzieren. Die PSE-Gene bzw. Nukleinsäuren können auch so manipuliert werden, dass die entsprechenden Mengen der verschiedenen Lipid- und Fettsäuremoleküle hergestellt werden. Dies kann eine einschneidende Wirkung auf die Lipidzusammensetzung der Membran der Zelle haben und erzeugt neue Öle zusätzlich zum Auftreten neusynthetisierter PUFAs. Da jeder Lipidtyp unterschiedliche physikalische Eigenschaften hat, kann eine Veränderung der Lipidzusammensetzung einer Membran die Membranfluidität erheblich verändern. Änderungen der Membranfluidität können sich auf den Transport von Molekülen über die Membran sowie auf die Unversehrtheit der Zelle auswirken, die beide eine entscheidende Wirkung auf die Produktion von Feinchemikalien besitzen. In Pflanzen können diese Änderungen überdies auch andere Merkmale, wie Toleranz gegenüber abiotischen und biotischen Stresssituationen, beeinflussen.

Biotische und abiotische Stresstoleranz ist ein allgemeines Merkmal, das man an ein breites Spektrum von Pflanzen, wie Mais, Weizen, Roggen, Hafer, Triticale, Reis, Gerste, Sojabohne, Erdnuss, Distel, Baumwolle, Raps und Canola, Maniok, Pfeffer, Sonnenblume und Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölpalme, Kokosnuss) und ausdauernde Gräser und Futterfeldfrüchte, vererben möchte. Diese Feldfrüchte sind als weitere erfindungsgemäße Ausführungsform auch bevorzugte Zielpflanzen für die Gentechnologie. Besonders bevorzugte erfindungsgemäße Pflanzen sind Ölfruchtpflanzen, wie Sojabohne, Erdnuss, Raps, Canola, Sonnenblume, Diestel, Bäume (Ölpalme, Kokosnuss) oder Feldfrüchte, wie Mais, Weizen, Roggen, Hafer, Triticale, Reis, Gerste, Alfalfa, oder Buschpflanzen (Kaffee, Kakao, Tee).

Im erfindungsgemäßen Verfahren werden isolierte Nukleinsäuremoleküle (z.B. cDNAs) eingesetzt, umfassend eine Nukleotidsequenz, die eine PSE oder biologisch aktive Teile davon kodiert. Bei besonders bevorzugten Ausführungsformen umfasst das Nukleinsäuremolekül eine der in SEQ ID NO:1 dargestellten Nukleotidsequenzen oder die kodierende Region oder ein Komplement einer dieser Nukleotidsequenzen. Bei anderen besonders bevorzugten Ausführungsformen umfasst das isolierte Nukleinsäuremolekül eine Nukleotidsequenz, die zu mindestens etwa 50 %, vorzugsweise mindestens etwa 60 %, stärker bevorzugt mindestens etwa 70%, 80 % oder 90 % und noch stärker bevorzugt mindestens etwa 95 %, 96 %, 97 %, 98 %, 99 % oder mehr homolog dazu ist, wobei unter Homologie im Sinne der Erfindung Identität zu verstehen ist. Bei anderen bevorzugten Ausführungsformen kodiert das isolierte Nukleinsäuremolekül eine der in der Sequenz SEQ ID NO:2 dargestellten Aminosäuresequenzen. Das PSE-Gen bzw. die Nukleinsäuresequenz besitzt auch mindestens eine der hier beschriebenen PSE-Aktivitäten.

Bei einer weiteren Ausführungsform kodiert das isolierte Nukleinsäuremolekül ein Protein oder einen Teil davon, wobei das Protein oder der Teil davon eine Aminosäuresequenz enthält, die ausreichend homolog zu einer Aminosäuresequenz der Sequenz SEQ ID NO:2 ist, dass das Protein oder der Teil davon eine PSE-Aktivität beibehält wobei unter Homologie im Sinne der Erfindung Identität zu verstehen ist. Vorzugsweise behält das Protein oder der Teil davon, das/der von dem Nukleinsäuremolekül kodiert wird, die Fähigkeit, am Stoffwechsel von zum Aufbau von Zellmembranen von Pflanzen notwendigen Verbindungen oder am Transport von Molekülen über diese Membranen teilzunehmen. Bei einer Ausführungsform ist das von dem Nukleinsäuremolekül kodierte Protein zu mindestens etwa 50 %, vorzugsweise mindestens etwa 60 % und stärker bevorzugt mindestens etwa 70 %, 80 % oder 90 % und am stärksten bevorzugt mindestens etwa 95 %, 96 %, 97 %, 98 %, 99 % oder mehr homolog zu einer Aminosäuresequenz der Sequenz SEQ ID NO:2. Bei einer weiteren bevorzugten Ausführungsform ist das Protein ein Volllängen- Phytophthora infestans-Protein, das im wesentlichen homolog zu einer gesamten Aminosäuresequenz der SEQ ID NO:2 (die von dem in SEQ ID NO:1 gezeigten offenen Leserahmen her rührt) ist.

Bei einer anderen bevorzugten Ausführungsform rührt das isolierte Nukleinsäuremolekül von Phytophthora infestans her und kodiert ein Protein (z.B. ein PSE-Fusionsprotein), das eine biologisch aktive Domäne enthält, die zu mindestens etwa 50 % oder mehr homolog (identisch) zu einer Aminosäuresequenz der Sequenz SEQ ID NO:2 ist und die Fähigkeit, am Stoffwechsel von Pilzen zum Aufbau von ungesättigten Fettsäuren notwendigen Verbindungen oder am Transport von Molekülen über Membranen teilzunehmen, beibehält oder zumindest eine der in der Tabelle I aufgeführten Aktivitäten besitzt, und umfasst auch heterologe Nukleinsäuresequenzen, die ein heterologes Polypeptid oder regulatorische Proteine kodieren.

Die Nukleinsäuren codieren für Proteine, die Fettsäuren ausgewählt aus der Gruppe C_{18:2}^{Δ9,12}, C_{18:3}^{Δ4,7,10}, C_{18:3}^{Δ5,8,11}, C_{18:3}^{Δ6,9,12}, C_{18:3}^{Δ7,10,13}, C_{18:3}^{Δ8,11,14}, C_{18:3}^{Δ9,12,15}, C_{18:4}^{Δ6,9,12,15}, C_{18:3}^{Δ5c,9,12} oder C_{16:}^{3Δ7,10,13} elongieren, während Fettsäuren wie C_{18:3}^{Δ5t,9,12}, C_{20:3}^{Δ8,11,14}, C_{20:4}^{Δ5,8,11,14} und C_{20:5Δ5,8,11,14,17} nicht verlängert werden. Das PSE Gen wurde heterolog in Hefe exprimiert und verschiedene mehrfach(poly)-ungesättigte Fettsäuren wurden einzeln gefüttert und von dem transformierten Hefen umgesetzt. Analysiert wurden Fettsäureprofile von transgenen Hefen mittels GLC. Der Prozentsatz von umgesetzten gefütterten Fettsäuren wurde folgendermaßen berechnet: mol% (Produkt)x100/[mol%(Edukt) + mol%(Produkt)].

Tabelle I gibt Fettsäuren wieder, die durch das oben genannte Enzym elongiert werden.

**Tabelle I: Fettsäuren (in mol%) von Hefezellen transformiert mit dem Plasmid pYES2 (Kontrolle) und pY2piPSE1. Gefüttert wurden 18:2, γ-18:3, α-18:3 oder 18:4 Fettsäuren. Die gesamte Lipidfraktion wurde transmethyliert und das Fettsäureprofil mittels GC bestimmt. Berechnung %-Elongation: 100 x mol% (Produkt)/[mol%(Edukt) + mol % (Produkt)]. Substrate und elongierte Fettsäuren werden im Fettdruck wiedergegeben.**

| | % of total fatty acids | | | | | | | |
|---|---|---|---|---|---|---|---|---|
| | PYES2 | | | | PY2piPSE1 | | | |
| Fatty acids | +18:2 | +γ-18:3 | +α-18:3 | +18:4 | +18:2 | +γ-18:3 | +α-18:3 | +18:4 |
| 16:0 | 13.7 | 16.4 | 14.3 | 19.5 | 9.8 | 9.8 | 7.3 | 10.6 |
| 16:1^{Δ9} | 8.8 | 6.5 | 3.4 | 7.8 | 9.9 | 4.1 | 1.3 | 2.3 |
| 18:0 | 6.9 | 9.9 | 11.3 | 13.3 | 8.6 | 12.3 | 14.4 | 19.4 |
| 18:1^{Δ9} | 9.9 | 10.5 | 6.0 | 13.6 | 15.3 | 11.8 | 5.6 | 8.4 |
| 18:2^{Δ9,12} | **60.7** | - | - | - | **41.0** | - | - | - |
| 18:3 ^{Δ6,9,12} | - | **56.7** | - | - | - | **51.1** | - | - |
| 18:3^{Δ9,12,15} | - | - | **64.7** | - | - | - | **65.1** | - |
| 18:4^{Δ6,9,12,15} | - | - | - | **45.7** | - | - | - | **48.3** |
| 20:2^{Δ11,14} | - | - | - | - | **2.1** | - | - | - |
| 20:3^{Δ8,11,14} | - | - | - | - | - | **11.0** | - | - |
| 20:3^{Δ11,14,17} | - | - | - | - | - | - | **6.2** | - |
| 20:4^{Δ8,11,14,17} | - | - | - | - | - | - | - | **11.0** |
| %elongation | **n.d.** | **n.d.** | **n.d.** | **n.d.** | **4.9** | **17.7** | **8.7** | **18.5** |

Das erfindungsgemäße Verfahren kann eingesetzt werden zur Modulation der Produktion von Feinchemikalien aus Mikroorganismen, wie Ciliaten, Pilzen, Hefen, Bakterien, Algen, und/oder Pflanzen, wie Mais, Weizen, Roggen, Hafer, Triticale, Reis, Gerste, Sojabohne, Erdnuss, Baumwolle, Diestel, Brassica-Arten, wie Raps, Diestel, Canola und Rübsen, Pfeffer, Sonnenblume, Borretsch, Nachtkerze und Tagetes, Solanaceen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwenden oder können eine indirekt Auswirkung haben, die dennoch zu einer Steigerung der Ausbeute, Produktion und/oder Effizienz der Produktion einer gewünschten Verbindung oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Feinchemikalien beeinflussen kann). Aspekte der Erfindung sind nachstehend weiter erläutert.

### I. Feinchemikalien und PUFAs

Der Begriff "Feinchemikalie" ist im Fachgebiet bekannt und umfasst Moleküle, die durch einen Organismus produziert worden sind und Anwendungen in verschiedenen Industrien finden, wie, aber nicht beschränkt auf, die pharmazeutische, Landwirtschafts-, Nahrungsmittel- und Kosmetik-Industrie. Diese Verbindungen umfassen Lipide, Fettsäuren, Cofaktoren und Enzyme usw. (wie z.B. beschrieben in Kuninaka, A. (1996) Nucleotides and related compounds, S. 561-612, in Biotechnology Bd. 6, Rehm et al., Hrsgb., VCH: Weinheim und darin enthaltenen Literaturstellen), Lipide, gesättigte und ungesättigte Fettsäuren (z.B. Arachidonsäure), Vitamine und Cofaktoren (wie beschrieben in Ullmann's Encyclopedia of Industrial Chemistry, Bd. A27, Vitamins, S. 443-613 (1996) VCH: Weinheim und darin enthaltenen Literaturstellen; und Ong, A.S., Niki, E., & Packer, L. (1995) Nutrition, Lipids, Health and Disease Proceedings of the UNESCO/Confederation of Scientific and Technological Associations in Malaysia and the Society for Free Radical Research - Asien, abgehalten am 1.-3. Sept. 1994 in Penang, Malaysia, AOCS Press (1995)), Enzyme und sämtliche anderen von Gutcho (1983) in Chemicals by Fermentation, Noyes Data Corporation, ISBN: 0818805086, und darin angegebenen Literaturstellen beschriebenen Chemikalien. Der Stoffwechsel und die Verwendungen bestimmter Feinchemikalien sind nachstehend weiter erläutert.

Die Kombination verschiedener Vorläufermoleküle und Biosyntheseenzyme führt zur Herstellung verschiedener Fettsäuremoleküle, was eine entscheidende Auswirkung auf die Zusammensetzung der Membran hat. Es kann angenommen werden, dass PUFAs nicht nur einfach in Triacylglycerin, sondern auch in Membranlipide eingebaut werden.

Die Synthese von Membranen ist ein gut charakterisierter Prozess, an dem eine Anzahl von Komponenten, einschließlich Lipiden als Teil der Bilayer-Membran, beteiligt sind. Die Produktion neuer Fettsäuren, wie PUFAs, kann daher neue Eigenschaften von Membranfunktionen innerhalb einer Zelle oder eines Organismus erzeugen.

Zellmembranen dienen einer Vielzahl von Funktionen in einer Zelle. Zuerst und in erster Linie grenzt eine Membran den Inhalt einer Zelle von der Umgebung ab, wodurch sie der Zelle Integrität verleiht. Membranen können auch als Schranken gegenüber dem Einstrom gefährlicher oder unerwünschter Verbindungen und auch gegenüber dem Ausstrom gewünschter Verbindungen dienen.

Detailliertere Beschreibungen und Beteiligungen von Membranen und die beteiligten Mechanismen siehe in: Bamberg, E., et al. (1993) Charge transport of ion pumps on lipid bilayer membranes, Q. Rev. Biophys. 26:1-25; Gennis, R.B. (1989) Pores, Channels and Transporters, in: Biomembranes, Molecular Structure and Function, Springer: Heidelberg, S. 270-322; und Nikaido, H., und Saier, H. (1992) Transport proteins in bacteria: common themes in their design, Science 258:936-942, und den in jeder dieser Literaturstellen enthaltenen Zitaten.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA entweder in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltene Literaturstellen).

Vorläufer für die PUFA-Biosynthese sind beispielsweise Palmitoleinsäure, Linol- und Linolensäure. Diese C₁₆- und/oder C₁₈-Kohlenstoff-Fettsäuren müssen auf C₂₀ und C₂₂ verlängert werden, damit Fettsäuren vom Eicosa- und Docosa-Kettentyp erhalten werden. Dieses Elongieren erfolgt vorteilhaft mit den erfindungsgemäßen Nukleinsäuren bzw. mit den durch diese Nukleinsäuren kodierten Proteine. Mit Hilfe verschiedener Desaturasen, wie Enzymen, welche Δ-6-Desaturase-, Δ-5- und Δ-4-Desaturaseaktivität aufweisen, können Arachidonsäure, Eicosapentensäure und Docosahexaensäure sowie verschiedene andere langkettige PUFAs erhalten, extrahiert und für verschiedene Zwecke bei Nahrungsmittel-, Futter-, Kosmetik- oder pharmazeutischen Anwendungen verwendet werden.

Zur Herstellung langkettiger PUFAs müssen, wie oben erwähnt, die mehrfach ungesättigten C₁₆- und/oder C₁₈-Fettsäuren um mindestens zwei Kohlenstoffatome durch die Enzymaktivität einer Elongase verlängert werden. Die oben genannten Nukleinsäuresequenzen kodieren Pflanzen-Elongasen, die C₁₆-und/oder C₁₈-Fettsäuren mit mindestens zwei Doppelbindungen in der Fettsäure um mindestens zwei Kohlenstoffatome verlängern können. Nach einer Elongationsrunde führt diese Enzymaktivität zu C₁₈- bzw. C₂₀-Fettsäuren, und nach zwei, drei und vier bzw. fünf Elongationsrunden zu C₂₂-, C₂₄- oder C₂₆-Fettsäuren. Mit diesen Elongasen können auch längere PUFAs synthetisiert werden. Die Aktivität der Elongasen führt zu C₂₀- und/oder C₂₂-Fettsäuren mit mindestens zwei Doppelbindungen im Fettsäuremolekül, vorzugsweise mit drei oder vier Doppelbindungen, besonders bevorzugt drei Doppelbindungen im Fettsäuremolekül. Nach der Elongation mit den Enzymen können weitere Desaturierungsschritte erfolgen. Daher führen die Produkte der Elongaseaktivität und der möglichen weiteren Desaturierung zu bevorzugten PUFAs mit höherem Desaturierungsgrad, wie Docosadiensäure, Arachidonsäure, ω6-Eicosatriendi-homo-γ-linolensäure, Eicosapentensäure, ω3-Eicosatriensäure, ω3-Eicosatetraensäure, Docosapentaensäure oder Docosahexaensäure. Substrate dieser Enzymaktivität sind zum Beispiel Taxolsäure, 6,9-Octadecadiensäure, Linolsäure, γ-Linolensäure, Pinolensäure, α-Linolensäure oder Stearidonsäure. Bevorzugte Substrate sind Linolsäure, γ-Linolensäure und/oder α-Linolensäure. Die C₁₆- und/oder C₁₈-Fettsäuren mit mindestens zwei Doppelbindungen in der Fettsäure können durch die Enzymaktivität in Form der freien Fettsäure oder in Form der Ester, wie Phospholipide, Glykolipide, Sphingolipide, Phosphoglyceride, Monoacylglycerin, Diacylglycerin oder Triacylglycerin, verlängert werden.

Ferner müssen Fettsäuren anschließend an verschiedene Modifikationen transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Weiterhin ändert sich durch die Expression der Nukleinsäuren in den verschiedenen Wirtsorganismen nicht nur die Zusammensetzung der Membranlipide insgesamt, sondern es verändert sich die Zusammensetzung aller Verbindungen in der Wirtszelle, die ungesättigte Fettsäuren enthalten, gegenüber den ursprünglichen Wirtszellen, die die Nukleinsäuren nicht oder nicht in diesen Mengen enthalten. Diese Veränderungen sind bei Wirtsorganismen beispielsweise Pflanzenzellen ausgeprägter, die die durch die Nukleinsäuren kodierten Proteine bzw. Enzyme natürlicherweise nicht enthalten.

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Vitamine, Cofaktoren und Nutrazeutika, wie PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden. Die Biosynthese dieser Moleküle in Organismen, die sie produzieren können, wie in Bakterien, ist im großen und ganzen charakterisiert worden (Ullmann's Encyclopedia of Industrial Chemistry, "Vitamins", Bd. A27, S. 443-613, VCH: Weinheim, 1996; Michal, G. (1999) Biochemical Pathways: An Atlas of Biochemistry and Molecular Biology, John Wiley & Sons; Ong, A.S., Niki, E., & Packer, L. (1995) "Nutrition, Lipids, Health and Disease" Proceedings of the UNESCO/Confederation of Scientific and Technological Associations in Malaysia and the Society for Free Radical Research Asia, abgehalten am 1.-3. Sept. 1994 in Penang, Malaysia, AOCS Press, Champaign, IL X, 374 S).

Die oben erwähnten Moleküle sind entweder selbst biologisch aktive Moleküle oder Vorstufen biologisch aktiver Substanzen, die entweder als Elektronenüberträger oder Zwischenprodukte bei einer Vielzahl von Stoffwechselwegen dienen. Diese Verbindungen haben neben ihrem Nährwert auch einen signifikanten industriellen Wert als Farbstoffe, Antioxidantien und Katalysatoren oder andere Verarbeitungshilfstoffe. (Einen Überblick über Struktur, Aktivität und industrielle Anwendungen dieser Verbindungen siehe z.B. in Ullmann's Encyclopedia of Industrial Chemistry, "Vitamins", Bd. A27, S. 443-613, VCH: Weinheim, 1996). Mehrfach ungesättigte Fettsäuren haben verschiedene Funktionen und gesundheitsfördernde Wirkungen, beispielsweise bei koronarer Herzerkrankung, Entzündungsmechanismen, Kinderernährung usw. Veröffentlichungen und Literaturstellen, einschließlich darin zitierter Literaturstellen, siehe in: Simopoulos, 1999, Am. J. Clin. Nutr. 70 (3. Suppl.):560-569, Takahata et al., Biosc. Biotechnol. Biochem. 1998, 62(11):2079-2085, Willich und Winther, 1995, Deutsche Medizinische Wochenschrift 120(7):229ff. Sie sind außerdem wichtige Ausgangsstoffe für die Synthese von Verbindungen, die wichtige biologische Vorgänge innerhalb des Organismus steuern. Sie finden deshalb beispielsweise in verschiedenen diätischen Lebensmitteln oder Medikamenten Anwendung.

### II. Elemente und Verfahren der Erfindung

Die vorliegende Erfindung beruht zumindest teilweise auf PSE-Nukleinsäure- und -Proteinmolekülen, welche eine Wirkung auf die Produktion von Zellmembranen oder Fettsäuren wie in Physcomitrella patens, Ceratodon purpureus und/oder Phytophthora infestans ausüben und beispielsweise die Bewegung von Molekülen über diese Membranen beeinflussen. Bei einer Ausführungsform nehmen die PSE-Moleküle am Stoffwechsel von zum Aufbau von Zellmembranen und/oder Fettsäuren in Organismen, wie Mikroorganismen und Pflanzen, notwendigen Verbindungen teil oder beeinflussen indirekt den Transport von Molekülen über diese Membranen. Bei einer bevorzugten Ausführungsform hat die Aktivität der PSE-Moleküle zur Regulation der Produktion von Membrankomponenten und des Membrantransports eine Auswirkung auf die Produktion der gewünschten Feinchemikalie durch diesen Organismus. Bei einer besonders bevorzugten Ausführungsform ist die Aktivität der PSE-Moleküle moduliert, so dass die Ausbeute, Produktion und/oder Effizienz der Produktion der Stoffwechselwege von Mikroorganismen oder Pflanzen, welche die PSEs regulieren, moduliert sind und die Effizienz des Transport von Verbindungen durch die Membranen verändert ist, was entweder direkt oder indirekt die Ausbeute, Produktion und/oder Effizienz der Produktion einer gewünschten Feinchemikalie durch Mikroorganismen und Pflanzen moduliert.

Der Begriff PSE oder PSE-Polypeptid umfasst Proteine, die am Stoffwechsel von zum Aufbau von Zellmembranen in Organismen, wie Mikroorganismen und Pflanzen, notwendigen Verbindungen oder am Transport von Molekülen über diese Membranen teilnehmen. Beispiele für PSEs sind in der SEQ ID NO:1 oder ihren Homologa, Derivaten oder Analoga offenbart. Die Begriffe PSE oder PSE-Nukleinsäuresequenz(en) umfassen Nukleinsäuresequenzen, die eine PSE kodieren und bei denen ein Teil eine kodierende Region und ebenfalls entsprechende 5'- und 3'-untranslatierte Sequenzbereiche ist. Beispiele für PSE-Gene sind die in SEQ ID NO:1 dargestellten. Die Begriffe Produktion oder Produktivität sind im Fachgebiet bekannt und beinhalten die Konzentration des Fermentationsproduktes (zum Beispiel der gewünschten Feinchemikalie), das in einer bestimmten Zeitspanne und einem bestimmten Fermentationsvolumen gebildet wird (z.B. kg Produkt pro Stunde pro Liter). Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und/oder stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und/oder stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Bei einer Ausführungsform können die PSE-Moleküle die Produktion eines gewünschten Moleküls, wie einer Feinchemikalie, in einem Mikroorganismus oder in Pflanzen modulieren. Es gibt eine Reihe von Mechanismen, durch die die Veränderung einer erfindungsgemäßen PSR die Ausbeute, Produktion und/oder Effizienz der Produktion einer Feinchemikalie aus einem Mikroorganismus- oder Pflanzenstamm, die dieses veränderte Protein enthalten, direkt beeinflussen kann. Die Anzahl oder Aktivität von PSEs, die am Transport von Feinchemikalienmolekülen innerhalb oder aus der Zelle beteiligt sind, kann erhöht werden, so dass größere Mengen dieser Verbindungen über Membranen transportiert werden, aus denen sie leichter gewonnen und ineinander umgewandelt werden. Ferner sind Fettsäuren, Triacylglycerine und/oder Lipide selbst wünschenswerte Feinchemikalien; durch Optimierung der Aktivität oder Steigern der Anzahl einer oder mehrerer PSEs, die an der Biosynthese dieser Verbindungen beteiligt sind, oder durch Stören der Aktivität einer oder mehrerer PSEs, die am Abbau dieser Verbindungen beteiligt sind, kann es möglich sein, die Ausbeute, Produktion und/oder Effizienz der Produktion von Fettsäure- und Lipidmolekülen aus Organismen, wie Mikroorganismen oder Pflanzen, zu erhöhen.

Die Mutagenese der Nukleinsäuren bzw. PSE-Gene kann auch PSEs mit veränderten Aktivitäten hervorbringen, welche die Produktion einer oder mehrerer gewünschter Feinchemikalien aus Mikroorganismen oder Pflanzen indirekt beeinflussen. Beispielsweise können erfindungsgemäße PSEs, die am Export von Abfallprodukten beteiligt sind, eine größere Anzahl oder höhere Aktivität aufweisen, so dass die normalen Stoffwechselabfälle der Zelle (deren Menge möglicherweise aufgrund der Überproduktion der gewünschten Feinchemikalie erhöht ist) effizient exportiert werden, bevor sie die Moleküle in der Zelle schädigen können (was die Lebensfähigkeit der Zelle herabsetzen würde) oder die Feinchemikalien-Biosynthesewege stören können (was die Ausbeute, Produktion oder Effizienz der Produktion einer gewünschten Feinchemikalie senken würde). Die relativ großen intrazellulären Mengen der gewünschten Feinchemikalie selbst können ferner für die Zelle toxisch sein, so dass man durch Steigern der Aktivität oder Anzahl von Transportern, die diese Verbindungen aus der Zelle exportieren können, die Lebensfähigkeit der Zelle in Kultur steigern kann, was wiederum zu einer größeren Anzahl an Zellen in der Kultur führt, welche die gewünschte Feinchemikalie produzieren. Die PSEs können auch so manipuliert werden, dass die entsprechenden Mengen unterschiedlicher Lipid- und Fettsäuremoleküle produziert werden. Dies kann eine erhebliche Auswirkung auf die Lipidzusammensetzung der Zellmembran haben. Da jeder Lipidtyp unterschiedliche physikalische Eigenschaften aufweist, kann eine Veränderung der Lipidzusammensetzung einer Membran die Membranfluidität signifikant verändern. Änderungen der Membranfluidität können den Transport von Molekülen über die Membran sowie die Integrität der Zelle beeinflussen, was jeweils eine erhebliche Auswirkung auf die Produktion von Feinchemikalien aus Mikroorganismen und Pflanzen in Fermentationskultur im großen Maßstab hat. Pflanzenmembranen verleihen spezifische Eigenschaften, wie Toleranz gegenüber Wärme, Kälte, Salz, Trockenheit sowie Toleranz gegen Pathogene, wie Bakterien und Pilze. Daher kann die Modulation der Membrankomponenten eine grundlegende Wirkung auf die Überlebensfähgikeit der Pflanzen unter den oben genannten Stressparametern haben. Dies kann über Änderungen in Signalkaskaden oder direkt über die veränderte Membranzusammensetzung erfolgen (siehe zum Beispiel: Chapman, 1998, Trends in Plant Science, 3(11):419-426) und Signalkaskaden (siehe Wang 1999, Plant Physiology, 120:645-651) oder die Kältetoleranz, wie offenbart in WO 95/18222, beeinflussen.

Die oben genannten, im erfindungsgemäßen Verfahren eingesetzten isolierten Nukleinsäuresequenzen sind im Genom eines Phytophthora infestans- Stamms enthalten, die beispielsweise über die Sammlungen wie ATCC oder DSM verfügbar sind. Die Nukleotidsequenz der isolierten Physcomitrella patenscDNA und die abgeleiteten Aminosäuresequenzen der Phytophthora infestans -PSEs sind in den SEQ ID NO:1 bzw. 2 gezeigt. Es wurden Computeranalysen durchgeführt, die diese Nukleotidsequenzen als Sequenzen klassifizierten und/oder identifizierten, die am Stoffwechsel von Zellmembrankomponenten beteiligte Proteine oder am Transport von Verbindungen über Zellmembranen bzw. Fettsäuren beteiligte Proteine kodieren. Ein EST mit der Datenbankeingabe-Nr. 08_ck19_b07 des Erfinders ist Teil der gezeigten Sequenz in SEQ ID NO:1. Inzwischen wurden diese ESTs umbenannt, was zu dem überarbeiteten Namen: pp001019019f führte. Auf analoge Weise wurde das partielle Polypeptid als pp001019019f bezeichnet. Das vollständige Fragment-Insert des ESTs pp001019019f wurde sequenziert und ergab die SEQ ID NO:1, welche die vollständige Sequenz von pp001019019f zeigt. Sie enthält eine vollständigen, funktionell aktiven Klon, von dem sich nach spezifischer Expression in Hefe herausstellte, dass er die gewünschte Substratspezifität aufwies, wie im Beispielteil gezeigt ist.

Die vorliegende Erfindung betrifft auch Verfahren, bei denen Proteine mit einer Aminosäuresequenz eingesetzt werden, die im wesentlichen homolog (= identisch) zu einer Aminosäuresequenz der SEQ ID NO:2 ist. Wie hier verwendet, ist ein Protein mit einer Aminosäuresequenz, die im wesentlichen homolog zu einer ausgewählten Aminosäuresequenz ist, zu mindestens etwa 50 % homolog zu der ausgewählten Aminosäuresequenz, z.B. der gesamten ausgewählten Aminosäuresequenz. Ein Protein mit einer Aminosäuresequenz, die im wesentlichen homolog zu einer ausgewählten Aminosäuresequenz ist, kann auch zu mindestens etwa 50 bis 60 %, vorzugsweise mindestens etwa 60 bis 70 % und stärker bevorzugt mindestens etwa 70 bis 80 %, 80 bis 90 % oder 90 bis 95 % und am stärksten bevorzugt mindestens etwa 96 %, 97 %, 98 %, 99 % oder mehr homolog zu einer ausgewählten Aminosäuresequenz sein.

Die PSE oder der biologisch aktive Teil oder das Fragment davon kann am Stoffwechsel von zum Aufbau von Zellmembranen in Mikroorganismen oder Pflanzen notwendigen Verbindungen oder am Transport von Molekülen über diese Membranen teilnehmen oder eine oder mehrere der zur Elongation von C₁₈-PUFAs benötigten Aktivitäten besitzen, so dass C₂₂- oder C₂₄-PUFAs sowie verwandte PUFAs erhalten werden.

Verschiedene Aspekte der Erfindung sind eingehender in den folgenden Unterabschnitten beschrieben.

### A. Isolierte Nukleinsäuremoleküle

Im erfindungsgemäßen Verfahren wird eingesetzt eine isolierte Nukleinsäure, umfassend eine Nukleotidsequenz, die ein Polypeptid kodiert, das C₁₆- und/oder C₁₈-Fettsäuren mit mindestens zwei Doppelbindungen in der Fettsäure verlängert, ausgewählt aus der Gruppe, bestehend aus
a) einer in SEQ ID NO:1 dargestellten Nukleinsäuresequenz,
b) einer Nukleinsäuresequenz, die gemäß dem degenerierten genetischen Code von der in SEQ ID NO:1 dargestellten Sequenz stammt,
c) Derivate der in SEQ ID NO:1 dargestellten Sequenz, die Polypeptide mit mindestens 50 % Homologie zu der Sequenz kodiert, die die Aminosäuresequenzen in SEQ ID NO:2 kodiert, wobei die Sequenz als C₁₆- oder C₁₈-Elongase wirkt-, wie andern Orts genauer beschrieben

Die oben genannte Nukleinsäure stammt von Organismen, wie Ciliaten, Pilzen, Algen oder Dinoflagellaten, die PUFAs synthetisieren können, vorzugsweise von Pflanzen oder Pilzen, besonders bevorzugt aus der Gattung Phytophthora und am stärksten bevorzugt von Phytophthora infestans.

Der Begriff "Nukleinsäuremolekül", wie hier verwendet, soll einzelsträngige oder doppelsträngige DNA-Moleküle (z.B. cDNA oder genomische DNA) und/oder RNA-Moleküle (z.B. mRNA) sowie DNA- oder RNA-Analoga, die mittels Nukleotidanaloga erzeugt werden, oder DNA/RNA-Hybride umfassen. Dieser Begriff umfasst zudem die am 3'- und am 5'-Ende des kodierenden Genbereichs gelegene untranslatierte Sequenz: mindestens etwa 100 Nukleotide der Sequenz stromaufwärts des 5'-Endes des kodierenden Bereichs und mindestens etwa 20 Nukleotide der Sequenz stromabwärts des 3'-Endes des kodierenden Genbereichs. Das Nukleinsäuremolekül kann einzelsträngig oder doppelsträngig sein, ist aber vorzugsweise doppelsträngige DNA. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte PSE-Nukleinsäuremolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt (z.B. eine Physcomitrella patens-Zelle) flankieren. Ein "isoliertes" Nukleinsäuremolekül, wie ein cDNA-Molekül, kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein oben genanntes Nukleinsäuremolekül, z.B. ein Nukleinsäuremolekül mit einer Nukleotidsequenz der SEQ ID NO:1 oder eines Teils davon, kann unter Verwendung molekularbiologischer Standardtechniken und der hier bereitgestellten Sequenzinformation isoliert werden. Beispielsweise kann eine Phytophthora infestans-cDNA aus einer P. infestans-Bank isoliert werden, indem die vollständige SEQ ID NO:1 oder ein Teil davon als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine vollständige Sequenz der SEQ ID NO:1 oder einen Teil davon, durch Polymerasekettenreaktion isolieren, wobei Oligonukleotidprimer, die auf der Basis dieser Sequenz oder von Teilen davon, insbesondere Regionen um His-Box-Motive, siehe Shanklin et al. (1994) Biochemistry 33, 12787-12794, erstellt wurden, verwendet werden (z.B. kann ein Nukleinsäuremolekül, umfassend die vollständigen Sequenz der SEQ ID NO:1 oder einen Teil davon, durch Polymerasekettenreaktion unter Verwendung von Oligonukleotidprimern isoliert werden, die auf der Basis dieser gleichen Sequenz der SEQ ID NO:1 erstellt worden sind). Zum Beispiel lässt sich mRNA aus Oomyzetenzellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St. Petersburg, FL) herstellen. Synthetische Oligonukleotidprimer zur Amplifizierung mittels Polymerasekettenreaktion lassen sich auf der Basis einer der in SEQ ID NO:1 gezeigten Nukleotidsequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer PSE-Nukleotidsequenz entsprechen, können durch Standard-Syntheseverfahren, beispielsweise mit einem automatischen DNA-Synthesegerät, hergestellt werden.

Die in SEQ ID NO:1 gezeigte cDNA umfasst Sequenzen, die PSEs kodieren, (d.h. den "kodierenden Bereich") sowie 5'-untranslatierte Sequenzen und 3'-untranslatierte Sequenzen. Alternativ kann das Nukleinsäuremolekül nur den kodierenden Bereich einer der Sequenzen in SEQ ID NO:1 umfassen oder kann ganze genomische Fragmente, die aus genomischer DNA isoliert sind, enthalten.

Bei einer weiteren bevorzugten Ausführungsform umfasst ein isoliertes Nukleinsäuremolekül ein Nukleinsäuremolekül, das ein Komplement einer der in SEQ ID NO:1 gezeigten Nukleotidsequenzen oder eines Teils davon ist. Ein Nukleinsäuremolekül, das zu einer der in SEQ ID NO:1 gezeigten Nukleotidsequenzen komplementär ist, ist eines, das zu einer der in SEQ ID NO:1 gezeigten Nukleotidsequenzen ausreichend komplementär ist, dass es mit einer der in SEQ ID NO:1 angegebenen Sequenzen hybridisieren kann, wodurch ein stabiler Duplex entsteht.

Homologa der neuen Elongase-Nukleinsäuresequenz mit der Sequenz SEQ ID NO:1 bedeutet beispielsweise allelische Varianten mit mindestens etwa 50 bis 60 %, vorzugsweise mindestens etwa 60 bis 70 %, stärker bevorzugt mindestens etwa 70 bis 80 %, 80 bis 90 % oder 90 bis 95 % und noch stärker bevorzugt mindestens etwa 95 %, 96 %, 97 %, 98 %, 99 % oder mehr Homologie zu einer in SEQ ID NO:1 gezeigten Nukleotidsequenzen oder ihren Homologa, Derivaten oder Analoga oder Teilen davon, wobei Homologie im Sinne der Erfindung Identität bedeutet. Bei einer weiteren bevorzugten Ausführungsform umfasst ein isoliertes erfindungsgemäßes Nukleinsäuremolekül eine Nukleotidsequenz, die an eine der in SEQ ID NO:1 gezeigten Nukleotidsequenzen oder einen Teil davon hybridisiert, z.B. unter stringenten Bedingungen hybridisiert. Allelische Varianten umfassen insbesondere funktionelle Varianten, die sich durch Deletion, Insertion oder Substitution von Nukleotiden aus/in der in SEQ ID NO:1 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Elongase besitzen, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 2 kodierten Protein.

Homologa der SEQ ID NO:1 bedeutet beispielsweise auch bakterielle, Pilz- und Pflanzenhomologa, verkürzte Sequenzen, einzelsträngige DNA oder RNA der kodierenden und nichtkodierenden DNA-Sequenz.

Homologa der SEQ ID NO:1 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die oben genannten Nukleinsäuremoleküle kodieren ein Protein oder einen Teil davon, das/der eine Aminosäuresequenz umfasst, die ausreichend homolog zu einer Aminosäuresequenz der SEQ ID NO:2 ist, dass das Protein oder der Teil davon die Fähigkeit, am Stoffwechsel von zum Aufbau von Zellmembranen in Mikroorganismen oder Pflanzen notwendigen Verbindungen oder am Transport von Molekülen über diese Membranen oder an der Fettsäuresynthese teilzunehmen, beibehält. Wie hier verwendet, betrifft der Begriff "ausreichend homolog" Proteine oder Teile davon, deren Aminosäuresequenzen eine minimale Anzahl identischer oder äquivalenter Aminosäurereste (z.B. einen Aminosäurerest mit einer ähnlichen Seitenkette, wie ein Aminosäurerest in einer der Sequenzen der SEQ ID NO:2) zu einer Aminosäuresequenz der SEQ ID NO:2 aufweisen, so dass das Protein oder der Teil davon am Stoffwechsel von zum Aufbau von Zellmembranen in Mikroorganismen oder Pflanzen notwendigen Verbindungen oder am Transport von Molekülen über diese Membranen teilnehmen kann. Proteinbestandteile dieser Stoffwechselwege für Membrankomponenten oder Membrantransportsysteme können, wie hier beschrieben, eine Rolle bei der Produktion und Sekretion einer oder mehrerer Feinchemikalien spielen. Beispiele für diese Aktivitäten sind hier ebenfalls beschrieben. Somit trägt die "Funktion einer PSE" entweder direkt oder indirekt zur Ausbeute, Produktion und/oder Effizienz der Produktion einer oder mehrerer Feinchemikalien bei. Beispiele für PSE-Substratspezifitäten der katalytischen Aktivität sind in Tabelle I angegeben.

Bei einer weiteren Ausführungsform des erfindungsgemäßen Verfahrens kodieren Derivate des Nukleinsäuremoleküls Proteine mit mindestens etwa 50 bis 60 %, vorzugsweise mindestens etwa 60 bis 70 % und stärker bevorzugt mindestens etwa 70 bis 80 %, 80 bis 90 %, 90 bis 95 % und am stärksten bevorzugt mindestens etwa 96 %, 97 %, 98 %, 99 % oder mehr Homologie zu einer vollständigen Aminosäuresequenz der SEQ ID NO:2. Die Homologie der Aminosäuresequenz wurde über den gesamten Sequenzbereich mit dem Programm PileUp (J. Mol. Evolution., 25, 351-360, 1987, Higgins et al., CABIOS, 5, 1989:151-153) bestimmt.

Teile von Proteinen, die von den PSE-Nukleinsäuremolekülen kodiert werden, sind vorzugsweise biologisch aktive Teile einer der PSEs. Wie hier verwendet, soll der Begriff "biologisch aktiver Teil einer PSE", einen Abschnitt, z.B. eine Domäne/ein Motiv, einer PSE umfassen, der am Stoffwechsel von zum Aufbau von Zellmembranen in Mikroorganismen oder Pflanzen notwendigen Verbindungen oder am Transport von Molekülen über diese Membranen teilnehmen kann oder an der Fettsäuresynthese beteiligt ist oder eine in Tabelle I angegebene Aktivität aufweist. Zur Bestimmung, ob eine PSE oder ein biologisch aktiver Teil davon am Stoffwechsel von zum Aufbau von Zellmembranen in Mikroorganismen oder Pflanzen notwendigen Verbindungen oder am Transport von Molekülen über diese Membranen teilnehmen kann, kann ein Test der enzymatischen Aktivität durchgeführt werden. Diese Testverfahren, wie eingehend in Beispiel 8 des Beispielteils beschrieben, sind dem Fachmann geläufig.

Zusätzliche Nukleinsäurefragmente, die biologisch aktive Abschnitte einer PSE kodieren, lassen sich durch Isolierung eines Teils der in SEQ ID NO:2 dargestellten Sequenz, Exprimieren des kodierten Abschnitt der PSE oder des Peptids (z.B. durch rekombinante Expression in vitro) und Bestimmen der Aktivität des kodierten Teils der PSE oder des Peptids herstellen.

Die Erfindung umfasst zudem den Einsatz von Nukleinsäuremolekülen, die sich von einer der in SEQ ID NO:1 gezeigten Nukleotidsequenzen (und Teilen davon) aufgrund des degenerierten genetischen Codes unterscheiden und somit die gleiche PSE kodieren wie diejenige, die von den in SEQ ID NO:1 gezeigten Nukleotidsequenzen kodiert wird. Bei einer anderen Ausführungsform hat ein isoliertes Nukleinsäuremolekül eine Nukleotidsequenz, die ein Protein mit einer in SEQ ID NO:2 gezeigten Aminosäuresequenz kodiert. Bei einer weiteren Ausführungsform kodiert das Nukleinsäuremolekül ein Vollängen- Phytophthora infestans-Protein, das zu einer Aminosäuresequenz der SEQ ID NO:2 (die von einem in SEQ ID NO:1 gezeigten offenen Leseraster kodiert wird) im wesentlichen homolog ist.

Zusätzlich zu den in SEQ ID NO:1 gezeigten Phytophthora infestans -PSE-Nukleotidsequenzen erkennt der Fachmann, dass DNA-Sequenzpolymorphismen, die zu Änderungen in den Aminosäuresequenzen der PSEs führen, innerhalb einer Population (z.B. der Phytophthora infestans-Population) existieren können. Diese genetischen Polymorphismen im PSE-Gen können zwischen Individuen innerhalb einer Population aufgrund von natürlicher Variation existieren. Wie hier verwendet, bedeuten die Begriffe "Gen" und "rekombinantes Gen" Nukleinsäuremoleküle mit einem offenen Leserahmen, der eine PSE, vorzugsweise eine Phytophthora infestans-PSE, kodiert. Diese natürlichen Varianten bewirken üblicherweise eine Varianz von 1 bis 5 % in der Nukleotidsequenz des PSE-Gens. Sämtliche und alle dieser Nükleotidvariationen und daraus resultierende Aminosäurepolymorphismen in PSE, die das Ergebnis natürlicher Variation sind und die funktionelle Aktivität von PSEs nicht verändern, sollen im Umfang der erfindungsgemäßen Verfahren enthalten sein.

Nukleinsäuremoleküle, die den natürlichen Varianten entsprechen, und nicht Phytophthora infestans-Homologa, -Derivate und -Analoga der Phytophthora infestans-PSE-cDNA können auf der Grundlage ihrer Homologie zu der hier offenbarten Phytophthora infestans-PSE-Nukleinsäure unter Verwendung der Phytophthora infestans-cDNA oder eines Teils davon als Hybridisierungssonde gemäß Standard-Hybridisierungstechniken unter stringenten Hybridisierungsbedingungen isoliert werden. Bei einer anderen Ausführungsform ist ein isoliertes Nukleinsäuremolekül mindestens 15 Nukleotide lang und hybridisiert unter stringenten Bedingungen mit dem Nukleinsäuremolekül, das eine Nukleotidsequenz der SEQ ID NO:1 umfasst. Bei anderen Ausführungsformen ist die Nukleinsäure mindestens 25, 50, 100, 250 oder mehr Nukleotide lang. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45_C, gefolgt von einem oder mehreren Waschschritten in 0,2 x SSC, 0,1 % SDS bei 50 bis 65_C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42_C und 58_C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42_C. Vorzugsweise sind die Hybridisierungsbedingungen für DNA:DNA-Hybride zum Beispiel 0,1 x SSC und 20_C bis 45_C, vorzugsweise zwischen 30_C und 45_C. Vorzugsweise sind die Hybridisierungsbedingungen für DNA:RNA-Hybride zum Beispiel 0,1 x SSC und 30_C bis 55_C, vorzugsweise zwischen 45_C und 55_C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können. Dem Fachmann ist bekannt, dass die Art und Zeit der Entwicklung der Hybridisierungsergebnisse einen Einfluss auf das Hybridisierungsergebnis hat. In einfachen Versuchen ist er in der Lage die Entwicklungsbedingungen so zu optimieren, dass die vorgenannte Hybridisierung zuverlässige sichere Ergebnisse liefert.

Vorzugsweise entspricht ein oben genanntes isoliertes Nukleinsäuremolekül, das unter stringenten Bedingungen an eine Sequenz der SEQ ID NO:1 hybridisiert, einem natürlich vorkommenden Nukleinsäuremolekül. Wie hier verwendet, betrifft ein "natürlich vorkommendes" Nukleinsäuremolekül ein RNA- oder DNA-Molekül mit einer Nukleotidsequenz, die in der Natur vorkommt (z.B. ein natürliches Protein kodiert). Bei einer Ausführungsform kodiert die Nukleinsäure eine natürliche vorkommendes Phytophthora infestans -PSE.

Zusätzlich zu natürlich vorkommenden Varianten der PSE-Sequenz, die in der Population existieren können, erkennt der Fachmann ferner, dass auch Änderungen durch Mutation in eine Nukleotidsequenz der SEQ ID NO:1 eingebracht werden können, was zu Änderungen der Aminosäuresequenz der kodierten PSE führt, ohne dass die Funktionsfähigkeit des PSE-Proteins beeinträchtigt wird. Beispielsweise lassen sich Nukleotidsusbtitutionen, die an "nicht-essentiellen" Aminosäureresten zu Aminosäuresubstitutionen führen, in einer Sequenz der SEQ ID NO:1 herstellen. Ein "nicht-essentieller" Aminosäurerest ist ein Rest, der sich in einer Wildtypsequenz einer der PSEs (SEQ ID NO:2) verändern lässt, ohne dass die Aktivität der PSE verändert wird, wohingegen ein "essentieller" Aminosäurerest für die PSE-Aktivität erforderlich ist. Andere Aminosäurereste (z.B. diejenigen, die in der Domäne mit PSE-Aktivität nicht konserviert oder lediglich semikonserviert sind) können jedoch für die Aktivität nicht essentiell sein und lassen sich somit wahrscheinlich verändern, ohne dass die PSE-Aktivität verändert wird.

Folglich betrifft ein weiterer Aspekt der Verfahren der Erfindung Nukleinsäuremoleküle, die PSEs kodieren, die veränderte Aminosäurereste enthalten, die für die PSE-Aktivität nicht essentiell sind. Diese PSEs unterscheiden sich in der Aminosäuresequenz von einer Sequenz in SEQ ID NO:2 und behalten dennoch zumindest eine der hier beschriebenen PSE-Aktivitäten. Das isolierte Nukleinsäuremolekül umfasst bei einer Ausführungsform eine Nukleotidsequenz, die ein Protein kodiert, wobei das Protein eine Aminosäuresequenz mit mindestens etwa 50 % Homologie zu einer Aminosäuresequenz der SEQ ID NO:2 umfasst und am Stoffwechsel von zum Aufbau von Zellmembranen in Phytophthora infestans notwendigen Verbindungen oder am Transport von Molekülen über diese Membranen teilnehmen kann oder am Fettsäurestoffwechsel beteiligt ist oder eine oder mehrere der in Tabelle I aufgeführten Aktivitäten besitzt. Das von dem Nukleinsäuremolekül kodierte Protein ist vorzugsweise mindestens etwa 50 bis 60 % homolog zu einer der Sequenzen in SEQ ID NO:2, stärker bevorzugt mindestens etwa 60 bis 70 % homolog zu einer der Sequenzen in SEQ ID NO:2, noch stärker bevorzugt mindestens etwa 70 bis 80 %, 80 bis 90 %, 90 bis 95 % homolog zu einer der Sequenzen in SEQ ID NO:2 und am stärksten bevorzugt mindestens etwa 96 %, 97 %, 98 % oder 99 % homolog zu einer der Sequenzen in SEQ ID NO:2.

Zur Bestimmung der prozentualen Homologie von zwei Aminosäuresequenzen (z.B. einer der Sequenzen der SEQ ID NO:2 und einer mutierten Form davon) oder von zwei Nukleinsäuren werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz (z.B. einer der Sequenzen der SEQ ID NO:2) durch den gleichen Aminosäurerest oder das gleiche Nukleotid wie die entsprechende Stelle in der anderen Sequenz (z.B. einer mutierten Form der aus SEQ ID NO:2 ausgewählten Sequenz) belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100).

Ein Nukleinsäuremolekül, das eine PSE kodiert, die zu einer Proteinsequenz der SEQ ID NO:2 homolog ist, kann durch Einbringen einer oder mehrerer Nukleotidsubstitutionen, - additionen oder -deletionen in eine Nukleotidsequenz der SEQ ID NO:1 erzeugt werden, so dass eine oder mehrere Aminosäuresubstitutionen, -additionen oder -deletionen in das kodierte Protein eingebracht werden. Mutationen können in eine der Sequenzen der SEQ ID NO:1 durch Standardtechniken, wie stellenspezifische (= site directed) Mutagenese und PCRvermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), betaverzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer PSE wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der PSE-kodierenden Sequenz eingebracht werden, z.B. durch Sättigungsmutagenese, und die resultierenden Mutanten können nach der hier beschriebenen PSE-Aktivität durchmustert werden, um Mutanten zu identifizieren, die PSE-Aktivität beibehalten. Nach der Mutagenese einer der Sequenzen der SEQ ID NO:1 kann das kodierte Protein rekombinant exprimiert werden, und die Aktivität des Proteins kann z.B. unter Verwendung der hier beschriebenen Tests (siehe Beispielteil) bestimmt werden.

### B. Genkonstrukt

In einer weiteren Ausführungsform der Verfahren der Erfindung wird ein Genkonstrukt eingesetzt, was eine isolierte Nukleinsäure bedeutet, die von einer Pflanze oder einem Pilz stammt und ein Polypeptid kodiert, das C₁₆- und/oder C₁₈-Fettsäuren mit mindestens zwei Doppelbindungen in der Fettsäure um mindestens zwei Kohlenstoffatome verlängert, oder die Gensequenz der SEQ ID NO:1, ihre Homologa, Derivate oder Analoga, die funktionsfähig mit einem oder mehreren Regulationssignalen, vorteilhafterweise zur Steigerung der Genexpression, verbunden sind. Beispiele für diese Regulationssequenzen sind Sequenzen, an die Induktoren oder Repressoren binden und so die Expression der Nukleinsäure regulieren. Zusätzlich zu diesen neuen Regulationssequenzen kann die natürliche Regulation dieser Sequenzen vor den eigentlichen Strukturgenen noch vorhanden sein und, wenn geeignet, genetisch modifiziert worden sein, so dass die natürliche Regulation ausgeschaltet worden ist und die Expression der Gene gesteigert worden ist. Das Genkonstrukt kann jedoch auch eine einfachere Struktur haben, d.h. dass keine zusätzlichen Regulationssignale vor der Sequenz SEQ ID NO:1 oder ihren Homologa inseriert worden sind und der natürliche Promotor mit seiner Regulation nicht deletiert worden ist. Statt dessen ist die natürliche Regulationssequenz so mutiert worden, dass keine Regulation mehr stattfindet und die Genexpression verstärkt ist. Das Genkonstrukt kann außerdem vorteilhafterweise eine oder mehrere sogenannte Enhancer-Sequenzen, die funktionsfähig mit dem Promotor verbunden sind und die gesteigerte Expression der Nukleinsäuresequenz ermöglichen, umfassen. Es ist auch möglich, am 3'-Ende der DNA-Sequenzen zusätzlich vorteilhafte Sequenzen zu inserieren, beispielsweise weitere Regulationselemente oder Terminatoren. Die Elongasegene können im Genkonstrukt in einer oder mehreren Kopien vorliegen. Es ist vorteilhaft für die Insertion weiterer Gene in Organismen, wenn weitere Gene im Genkonstrukt vorliegen.

Vorteilhafte Regulationssequenzen für das neue Verfahren liegen beispielsweise in Promotoren vor, wie dem cos-, tac-, trp-, tet-, trp-tet-, 1pp-, lac-, lpp-lac-, lacI^{q-}, T7-, T5-, T3-, gal-, trc-, ara-, SP6-, λ-P_{R}- oder λ-P_{L}-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenolinduzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodenspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der usp-, LEB4-, Phaseolin- oder Napin-Promotor. Weitere besonders vorteilhafte Promotoren sind samenspezifische Promotoren, die für Monokotyledonen oder Dikotyledonen verwendet werden können und in US 5,608,152 (Napin-Promotor aus Raps), WO 98/45461 (Phaseolin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LEB4-Promotor aus Leguminosen) beschrieben sind, wobei sich diese Promotoren für Dikotyledonen eignen. Die folgenden Promotoren eignen sich beispielsweise für Monokotyledonen lpt-2- oder 1pt-1-Promotor aus Gerste (WO 95/15389 und WO 95/23230), Hordein-Promotor aus und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich synthetische Promotoren zu verwenden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Die inserierten Gene können ihren eigenen Promotor haben oder auch unter der Kontrolle des Promotors der Sequenz SEQ ID NO:1 oder seiner Homologa stehen.

Das Genkonstrukt umfasst vorteilhafterweise zur Expression der anderen vorliegenden Gene weitere 3'- und/oder 5'-terminale Regulationssequenzen zur Steigerung der Expression, die in Abhängigkeit vom gewählten Wirtsorganismus und dem Gen oder den Genen für die optimale Expression ausgewählt werden.

Diese Regulationssequenzen sollen, wie oben erwähnt, die spezifische Expression der Gene und die Proteinexpression ermöglichen. Dies kann je nach dem Wirtsorganismus beispielsweise bedeuten, dass das Gen nur nach Induktion exprimiert oder überexprimiert wird oder dass es sofort exprimiert und/oder überexprimiert wird.

Die Regulationssequenzen oder -faktoren können außerdem vorzugsweise eine vorteilhafte Wirkung auf die Expression der eingebrachten Gene haben und diese somit steigern. Auf diese Weise ist es möglich, dass die Regulationselemente unter Verwendung starker Transkriptionssignale, wie Promotoren und/oder Enhancer, vorteilhafterweise auf Transkriptionsebene verstärkt werden. Es ist jedoch weiterhin auch möglich, die Translation zum Beispiel durch Verbesserung der mRNA-Stabilität zu verstärken.

### C. Rekombinante Expressionsvektoren und Wirtszellen

Ein weiterer Aspekt der Verfahren der Erfindung betrifft den Einsatz von Vektoren, vorzugsweise Expressionsvektoren, die eine Nukleinsäure enthalten, die eine PSE (oder einen Teil davon) kodieren. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung und episomale Säugervektoren). Andere Vektoren (z.B. nicht-episomale Säugervektoren) werden beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren (z.B. replikationsdefiziente Retroviren, Adenoviren und adenoverwandte Viren), die ähnliche Funktionen ausüben, umfassen. Ferner soll der Begriff Vektor auch andere Vektoren, die dem Fachmann bekannt sind, wie Phagen, Viren, wie SV40, CMV, Baculovirus, Adenovirus, Transposons, IS-Elemente, Phasmide, Phagemide, Cosmide, lineare oder zirkuläre DNA, umfassen.

Die oben genannten rekombinanten Expressionsvektoren umfassen eine Nukleinsäure oder ein Genkonstrukt in einer Form, die sich zur Expression der Nukleinsäure in einer Wirtszelle eignet, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann. Die erfindungsgemäßen Expressionsvektoren können in Wirtszellen eingebracht werden, um dadurch Proteine oder Peptide, einschließlich Fusionsproteinen oder -peptiden, herzustellen, die von den Nukleinsäuren, wie hier beschrieben, kodiert werden (z.B. PSEs, mutante Formen von PSEs, Fusionsproteine usw.).

Die rekombinanten Expressionsvektoren können zur Expression von PSEs in prokaryotischen oder eukaryotischen Zellen gestaltet sein. Beispielsweise können PSE-Gene in bakteriellen Zellen, wie C. glutamicum, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Pseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) oder nicht-humanen Säugerzellen exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Fusionsvektoren fügen eine Reihe von Aminosäuren an ein darin kodiertes Protein an, gewöhnlich am Aminoterminus des rekombinanten Proteins, aber auch am C-Terminus oder fusioniert innerhalb geeigneter Bereiche in den Proteinen. Diese Fusionsvektoren haben gewöhnlich drei Aufgaben: 1) die Verstärkung der Expression von rekombinantem Protein; 2) die Erhöhung der Löslichkeit des rekombinanten Proteins und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so dass die Abtrennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die PSE-kodierende Sequenz in einen pGEX-Expressionsvektor kloniert, so dass ein Vektor erzeugt wird, der ein Fusionsprotein kodiert, das vom N-Terminus zum C-Terminus GST-Thrombin-Spaltstelle-X-Protein umfasst. Das Fusionsprotein kann durch Affinitätschromatographie unter Verwendung von Glutathion-Agarose-Harz gereinigt werden. Rekombinante PSE, die nicht an GST fusioniert ist, kann durch Spaltung des Fusionsproteins mit Thrombin gewonnen werden.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-φ10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 φ1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III¹¹³-B1, ?gt11 or pBdCI, in Streptomyces pIJ101, pIJ364, pIJ702 oder pIJ361, in Bacillus pUB110, pC194 oder pBD214, in Corynebacterium pSA77 oder pAJ667.

Eine Strategie zur Maximierung der Expression von rekombinantem Protein ist die Expression des Proteins in einem Wirtsbakterium, dessen Fähigkeit zur proteolytischen Spaltung des rekombinanten Proteins gestört ist (Gottesman, S., Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so dass die einzelnen Codons für jede Aminosäure diejenigen sind, die vorzugsweise in einem zur Expression ausgewählten Bakterium, wie C. *glutamicum,* verwendet werden (Wada et al. (1992) Nucleic Acids Res. 20:2111-2118). Diese Veränderung der erfindungsgemäßen Nukleinsäuresequenzen erfolgt durch Standard-DNA-Synthesetechniken.

Bei einer weiteren Ausführungsform ist der PSE-Expressionsvektor ein Hefe-Expressionsvektor. Beispiele für Vektoren zur Expression in der Hefe S. cerevisiae umfassen pYepSec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise 2∝M, pAG-1, YEp6, YEp13 oder pEMBLYe23.

Alternativ können die PSEs in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018).

Bei einer weiteren Ausführungsform können die erfindungsgemäßen PSEs in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-t-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss funktionsfähig mit einem geeigneten Promotor verbunden sein, der die Genexpression auf rechtzeitige, zell- oder gewebespezifische Weise durchführt. Bevorzugt sind Promotoren, welche die konstitutive Expression herbeiführen (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alphaamylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder 1pt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Ein rekombinanter Expressionsvektor kann in eine Wirtszelle eingebracht werden. Die Begriffe "Wirtszelle", "rekombinante Wirtszelle" und "transgene Wirtszelle" werden hier untereinander austauschbar verwendet. Selbstverständlich betreffen diese Begriffe nicht nur die bestimmte Zielzelle, sondern auch die Nachkommen oder potentiellen Nachkommen dieser Zelle. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch immer noch vom Umfang des Begriffs, wie hier verwendet, umfasst.

Eine Wirtszelle kann eine prokaryotische oder eukaryotische Zelle sein. Zum Beispiel kann eine PSE in Bakterienzellen, wie C. glutamicum, Insektenzellen, Pilzzellen oder Säugerzellen (wie Chinesischer Hamster-Ovarzellen (CHO) oder COS-Zellen), Algen, Ciliaten, Pflanzenzellen, Pilzen oder anderen Mikroorganismen, wie C. glutamicum, exprimiert werden. Andere geeignete Wirtszellen sind dem Fachmann geläufig.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Zur Erzeugung eines homolog rekombinierten Mikroorganismus wird ein Vektor hergestellt, der zumindest einen Abschnitt eines PSE-Gens enthält, in den eine Deletion, Addition oder Substitution eingebracht worden ist, um dadurch das PSE-Gen zu verändern, z.B. funktionell zu disrumpieren. Dieses PSE-Gen ist vorzugsweise ein Physcomitrella patens- oder Phytophthora infestans-PSE-Gen, es kann jedoch ein Homologon oder Analogon aus einer verwandten Pflanze oder sogar aus einer Säuger-, Hefe- oder Insektenquelle verwendet werden. Bei einer bevorzugten Ausführungsform ist der Vektor so gestaltet, dass das endogene PSE-Gen bei homologer Rekombination funktionell disrumpiert wird (d.h. nicht länger ein funktionelles Protein kodiert, auch als Knock-out-Vektor bezeichnet). Alternativ kann der Vektor so gestaltet sein, dass das endogene PSE-Gen bei homologer Rekombination mutiert oder anderweitig verändert wird, aber immer noch ein funktionelles Protein kodiert (z.B. kann der stromaufwärts gelegene regulatorische Bereich so verändert sein, dass dadurch die Expression der endogenen PSE verändert wird). Zur Erzeugung einer Punktmutation über homologe Rekombination können auch als Chimeraplastie bekannte DNA-RNA-Hybride verwendet werden, die aus Cole-Strauss et al., 1999, Nucleic Acids Research 27(5):1323-1330 und Kmiec, Gene therapy, 19999, American Scientist, 87(3):240-247 bekannt sind.

Im Vektor für die homologe Rekombination ist der veränderte Abschnitt des PSE-Gens an seinem 5'- und 3'-Ende von zusätzlicher Nukleinsäure des PSE-Gens flankiert, so dass homologe Rekombination zwischen dem exogenen PSE-Gen, das auf dem Vektor vorliegt, und einem endogenen PSE-Gen in einem Mikroorganismus oder einer Pflanze möglich ist. Die zusätzliche flankierende PSE-Nukleinsäure ist für eine erfolgreiche homologe Rekombination mit dem endogenen Gen hinreichend lang. Gewöhnlich sind im Vektor mehrere hundert Basenpaare bis zu Kilobasen flankierende DNA (sowohl am 5'- als auch am 3'-Ende) enthalten (eine Beschreibung von Vektoren zur homologen Rekombination siehe z.B. in Thomas, K.R., und Capecchi, M.R. (1987) Cell 51:503 oder der Rekombination in Physcomitrella patens auf cDNA-Basis in Strepp et al., 1998, Proc. Natl. Acad. Sci. USA 95 (8):4368-4373). Der Vektor wird in einen Mikroorganismus oder eine Pflanzenzelle (z.B. mittels Polyethylenglycol-vermittelter DNA) eingebracht, und Zellen, in denen das eingebrachte PSE-Gen mit dem endogenen PSE-Gen homolog rekombiniert ist, werden unter Verwendung im Fachgebiet bekannter Techniken selektiert.

Bei einer anderen Ausführungsform können rekombinante Mikroorganismen, hergestellt werden, die ausgewählte Systeme enthalten, welche eine regulierte Expression des eingebrachten Gens ermöglichen. Der Einschluss eines PSE-Gens in einem Vektor, wobei es unter die Kontrolle des lac-Operons gebracht wird, ermöglicht z.B. die Expression des PSE-Gens nur in Gegenwart von IPTG. Diese Regulationssysteme sind im Fachgebiet bekannt.

In Pflanzen kann zusätzlich ein alternatives Verfahren durch direkten Transfer von DNA in sich entwickelnde Blüten über Elektroporation oder Gentransfer mittels Agrobacterium angewendet werden.

Besonders bevorzugt sind Pflanzen, wie Ölfruchtpflanzen, die große Mengen an Lipidverbindungen enthalten, wie Raps, Nachtkerze, Canola, Erdnuss, Lein, Soja, Diestel, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte sind Ölfruchtpflanzen, wie Soja, Erdnuss, Raps, Canola, Sonnenblume, Safflor, Bäume (Ölpalme, Kokosnuss).

"Transgen" meint bezüglich zum Beispiel einer Nukleinsäuresequenz, einer Expressionskassette (= Genkonstrukt) oder einem Vektor enthaltend besagte Nukleinsäuresequenz oder einem Organismus transformiert mit den oben genannten Nukleinsäuresequenzen, Expressionskassette oder Vektor alle solche durch gentechnische Methoden zustandegekommene Konstruktionen, in denen sich entweder
a) die erfindungsgemäße Nukleinsäuresequenz, oder
b) eine mit der erfindungsgemäßen Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der erfindungsgemäßen Nukleinsäuresequenz mit dem entsprechenden PSE-Gen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

### D. Erfindungsgemäße Verwendungen und Verfahren

Die hier beschriebenen Nukleinsäuremoleküle, Proteine, Proteinhomologa, Fusionsproteine, Vektoren und Wirtszellen können einem Verfahren nach Anspruch 1 verwendet werden÷.

Somit betrifft die Erfindung ein Verfahren zur Produktion von PUFAs, wobei das Verfahren das Züchten eines Mikro organismus oder einer Pflanze, der eine Nukleinsäure, ein Genkonstrukt oder einen Vektor umfasst, welche ein Polypeptid kodieren, das C₁₆- und/oder C₁₈-Fettsäuren mit mindestens zwei Doppelbindungen im Fettsäuremolekül um mindestens zwei Kohlenstoffatome unter Bedingungen, unter denen PUFAs in dem Organismus produziert werden, verlängert, umfasst. Durch dieses Verfahren hergestellte PUFAs lassen sich durch Ernten der Organismen entweder aus der Kultur, in der sie wachsen, oder von dem Feld, Aufbrechen und/oder Extrahieren des geernteten Materials mit einem organischen Lösungsmittel isolieren. Aus diesem Lösungsmittel kann das Öl, das Lipide, Phospholipide, Sphingolipide, Glycolipide, Triacylglycerine und/oder freie Fettsäuren mit höherem Gehalt an PUFAs enthält, isoliert werden. Durch basische oder saure Hydrolyse der Lipide, Phospholipide, Sphingolipide, Glycolipide, Triacylglycerine können die freien Fettsäuren mit höherem Gehalt an PUFAs isoliert werden. Ein höherer Gehalt an PUFAs bedeutet mindestens 5 %, vorzugsweise 10 %, besonders bevorzugt 20 %, ganz besonders bevorzugt 40 % mehr PUFAs als der ursprüngliche Organismus beispielsweise ein Oomyzet wie Phytophthora oder einer Pflanze wie einer Ölfruchtpflanze, der/die keine zusätzliche Nukleinsäure, die die erfindungsgemäße Elongase kodiert, besitzt. Weiterhin besitzten die vorgenannten Öle, Lipide, Phospholipide, Sphingolipide, Glycolipide, Triacylglycerine und/oder freie Fettsäuren mit höherem Gehalt an PUFAs eine andere Zusammensetzung als die Zusammensetzung der Ausgangsorganismen. Dies gilt insbesondere für Pflanzen, die längerkettige mehrfach ungesättigte C₂₀- oder C₂₂-Fettsäuren wie DHA, EPA oder ARA natürlicherweise nicht enthalten.

Vorzugsweise sind die durch dieses Verfahren produzierten PUFAs C₂₀- oder C₂₂-Fettsäuremoleküle mit mindestens zwei Doppelbindungen im Fettsäuremolekül, vorzugsweise drei oder vier Doppelbindungen, besonders bevorzugt drei Doppelbindungen. Diese C₂₀- oder C₂₂-Fettsäuremoleküle lassen sich aus dem Organismus in Form eines Öls, Lipids oder einer freien Fettsäure isolieren.

### Beispiele

### Beispiel 1: Allgemeine Verfahren

### a) Allgemeine Klonierungsverfahren:

Klonierungsverfahren, wie beispielsweise Restriktionsspaltungen, Agarosegelelektrophorese, Reinigung von DNA-Fragmenten, Transfer von Nukleinsäuren auf Nitrocellulose- und Nylonmembranen, Verbindung von DNA-Fragmenten, Transformation von Escherichia coli- und Hefe-Zellen, Züchtung von Bakterien und Sequenzanalyse rekombinanter DNA, wurden durchgeführt wie beschrieben in Sambrook et al. (1989) (Cold Spring Harbor Laboratory Press: ISBN 0-87969-309-6) oder Kaiser, Michaelis und Mitchell (1994) "Methods in Yeast Genetics" (Cold Spring Harbor Laboratory Press: ISBN 0-87969-451-3).

### b) Chemikalien

Die verwendeten Chemikalien wurden, wenn im Text nicht anders angegeben, in p. A.-Qualität von den Firmen Fluka (Neu-Ulm), Merck (Darmstadt), Roth (Karlsruhe), Serva (Heidelberg) und Sigma (Deisenhofen) bezogen. Lösungen wurden unter Verwendung von reinem pyrogenfreiem Wasser, im nachstehenden Text als H₂O bezeichnet, aus einer Milli-Q-Wassersystem-Wasserreinigungsanlage (Millipore, Eschborn) hergestellt. Restriktionsendonukleasen, DNA-modifizierende Enzyme und molekularbiologische Kits wurden bezogen von den Firmen AGS (Heidelberg), Amersham (Braunschweig), Biometra (Göttingen), Boehringer (Mannheim), Genomed (Bad Oeynhausen), New England Biolabs (Schwalbach/Taunus), Novagen (Madison, Wisconsin, USA), Perkin-Elmer (Weiterstadt), Pharmacia (Freiburg), Qiagen (Hilden) und Stratagene (Amsterdam, Niederlande). Wenn nicht anders angegeben, wurden sie nach den Anweisungen des Herstellers verwendet.

### Beispiel 2: Konstruktion der cDNA-Bank

Zur Konstruktion der cDNA-Bank wurde die Erststrangsynthese unter Verwendung von Reverser Transkriptase aus Maus-Leukämie-Virus (Roche, Mannheim, Deutschland) und Oligo-d(T)-Primern, die Zweitstrangsynthese durch Inkubation mit DNA-Polymerase I, Klenow-Enzym und RNAse H-Spaltung bei 12_C (2 Std.), 16_C (1 Std.) und 22_C (1 STd.) erzielt. Die Reaktion wurde durch Inkubation bei 65_C (10 min) gestoppt und anschließend auf Eis überführt. Doppelsträngige DNA-Moleküle wurde mit T4-DNA-Polymerase (Roche, Mannheim) bei 37_C (30 min) mit glatten Enden versehen. Die Nukleotide wurden durch Phenol/Chloroform-Extraktion und Sephadex-G50-Zentrifugiersäulen entfernt. EcoRI-Adapter (Pharmacia, Freiburg, Deutschland) wurden mittels T4-DNA-Ligase (Roche, 12_C, über Nacht) an die cDNA-Enden ligiert und durch Inkubation mit Polynukleotidkinase (Roche, 37_C, 30 min) phosphoryliert. Dieses Gemisch wurde der Trennung auf einem Low-Melting-Agarose-Gel unterworfen. DNA-Moleküle über 300 Basenpaaren wurden aus dem Gel eluiert, Phenol-extrahiert, auf Elutip-D-Säulen (Schleicher und Schüll, Daßel, Deutschland) konzentriert und an Vektorarme ligiert und in lambda-ZAPII-Phagen oder lambda-ZAP-Express-Phagen unter Verwendung des Gigapack Gold-Kits (Stratagene, Amsterdam, Niederlande) verpackt, wobei Material des Herstellers verwendet und seine Anweisungen befolgt wurden.

### Beispiel 3: DNA-Sequenzierung und Computeranalyse

cDNA-Banken, wie im Beispiel 4 beschrieben, wurden zur DNA-Sequenzierung nach Standardverfahren, insbesondere durch das Kettenterminationsverfahren unter Verwendung des ABI PRISM Big Dye Terminator Cycle Sequencing Ready Reaction-Kit (Perkin-Elmer, Weiterstadt, Deutschland), verwendet. Die Zufallssequenzierung wurde anschließend an die präparative Plasmidgewinnung aus cDNA-Banken über in vivo-Massenausschnitt und Retransformation von DH10B auf Agarplatten durchgeführt (Einzelheiten zu Material und Protokoll von Stratagene, Amsterdam, Niederlande). Plasmid-DNA wurde aus über Nacht gezüchteten E. coli-Kulturen, die in Luria-Brühe mit Ampicillin (siehe Sambrook et al. (1989) (Cold Spring Harbor Laboratory Press: ISBN 0-87969-309-6)) gezüchtet worden waren, an einem Quiagen-DNA-Präparations-Roboter (Quiagen, Hilden) nach den Protokollen des Herstellers präpariert. Sequenzierprimer mit den folgenden Nukleotidsequenzen wurden verwendet:
5'-CAGGAAACAGCTATGACC-3'
5'-CTAAAGGGAACAAAAGCTG-3'
5'-TGTAAAACGACGGCCAGT-3'

Die Sequenzen wurden unter Verwendung des Standard-Softwarepakets EST-MAX, das kommerziell von Bio-Max (München, Deutschland) geliefert wird, Prozessiert und kommentiert. Ein Klon mit schwachen Homologien zu bekannten Elongasen wurde eingehender charakterisiert.

### Beispiel 4: Identifizierung des PSE1-Gens aus P. infestans und Analyse des cDNA-Klons PiPSE1

Eine EST-Sequenz (Datenbankeintrag: PI001002014r) wurde aufgrund schwacher Homologie mit bekannten Elongasen unter anderen Kandidatengenen als Zielgen in Betracht gezogen.

Für den Sequenzvergleich wurde das Programm BESTFIT verwendet, d.h. die BLOSUM-Aminosäuresubstitutions-Matrizen, wobei auf Henikoff, S., und Henikoff. J.G. (1992), Amino acid substitution matrices from protein blocks, Proc. Natl. Acad. Sci. USA 89:10915-10919, verwiesen wird.

Die Sequenz des Klons mit der Datenbank-Nr. PI001002014r wurde für den Vergleich mit der elol-Peptidsequenz aus Hefe verwendet. Da der neue P. infestans Klon nicht vollständig war, wurde ausgehed von der P. infestans cDNA Bank der korespondierende full length Klon isoliert (PiPSEI). Hierzu wurde eine Digoxigeninmarkierte Sonde durch PCR mittels des PCR DIG synthesis kits (Roche) hergestellt, wobei PI001002014 als Template diente. Für die PCR wurden folgende Primer verwendet:

| | |
|---|---|
| PI-DIGf: | cacaccatcatgtacacttactac |
| PI-DIGr: | caacttcttcttcgattcctccac |

Die isolierte markierte Sonde wurde für ein Screening der P. infestans cDNA Bank verwendet (entsprechend den Angaben des Herstellers, Stratagene). Ein Fragment von 1046 bp Länge wurde isoliert und als PiPSE1 bezeichnet. Das Offene-Leseraster ist 837 bp lang und kodiert für ein Protein von 278 Aminosäuren mit einer berechneten Molekularen Masse von 32,1 kDa. Sequenzvergleiche ergaben folgende Sequenzidentitäten, bzw. - ähnlichkeiten: 26 % / 43 % mit der Physcomitrella patens PSE1p, 23 % / 37 % mit der humanen HELOp, 21 % / 41 % mit der GLELOp aus Mortierella alpina und 17 % / 36 % mit der Elongase aus C. elegans.

### Beispiel 5: Identifikation von Genen mittels Hybridisierung

Gensequenzen lassen sich zur Identifikation homologer oder heterologer Gene aus cDNA- oder genomischen Banken verwenden.

Homologe Gene (d.h. Volllängen-cDNA-Klone, die homolog sind, oder Homologa) lassen sich über Nukleinsäurehybridisierung unter Verwendung von beispielsweise cDNA-Banken isolieren: Je nach der Häufigkeit des Gens von Interesse wurden 100000 bis zu 1000000 rekombinante Bakteriophagen plattiert und auf eine Nylonmembran überführt. Nach der Denaturierung mit Alkali wurde die DNA auf der Membran z.B. durch UV-Vernetzung immobilisiert. Die Hybridisierung erfolgte bei hoch-stringenten Bedingungen. In wässriger Lösung wurden die Hybridisierung und die Waschschritte bei einer Ionenstärke von 1 M NaCl und einer Temperatur von 68_C durchgeführt. Hybridisierungssonden wurden z.B. durch Markierung mittels radioaktiver (³²P-) Nicktranskription (High Prime, Roche, Mannheim, Deutschland) hergestellt. Die Signale wurden mittels Autoradiographie nachgewiesen.

Partiell homologe oder heterologe Gene, die verwandt, aber nicht identisch sind, lassen sich analog zum oben beschriebenen Verfahren unter Verwendung niedrig-stringenter Hybridisierungs- und Waschbedingungen identifizieren. Für die wässrige Hybridisierung wurde die Ionenstärke gewöhnlich bei 1 M NaCl gehalten, wobei die Temperatur nach und nach von 68 auf 42_C gesenkt wurde.

Die Isolation von Gensequenzen, die nur zu einer einzelnen Domäne von beispielsweise 10 bis 20 Aminosäuren Homologien aufweisen, lässt sich unter Verwendung synthetischer, radioaktiv markierter Oligonukleotidsonden durchführen. Radioaktiv markierte Oligonukleotide wurden mittels Phosphorylierung des 5'-Endes zweier komplementärer Oligonukleotide mit T4-Polynukleotidkinase hergestellt. Die komplementären Oligonukleotide wurden aneinander hybridisiert und ligiert, so dass Konkatemere entstanden. Die doppelsträngigen Konkatemere wurde beispielsweise durch Nicktranskription radioaktiv markiert. Die Hybridisierung erfolgte gewöhnlich bei niedrig-stringenten Bedingungen unter Verwendung hoher Oligonukleotidkonzentrationen.

Oligonukleotid-Hybridisierungslösung:
6 x SSC
0,01 M Natriumphosphat
1 mM EDTA (pH 8)
0,5 % SDS
100 ∝g/ml denaturierte Lachssperma-DNA
0,1 % fettarme Trockenmilch

Während der Hybrdidisierung wurde die Temperatur schrittweise auf 5 bis 10_C unter die berechnete Oligonukleotid-Tm oder bis auf Raumtemperatur (bedeutet RT = ~ 23_C in allen Experimenten, wenn nicht anders angegeben) gesenkt, gefolgt von Waschschritten und Autoradiographie. Das Waschen wurde mit extrem niedriger Stringenz durchgeführt, zum Beispiel 3 Waschschritte unter Verwendung von 4 x SSC. Weitere Einzelheiten sind wie von Sambrook, J., et al. (1989), "Molecular Cloning: A Laboratory Manual", Cold Spring Harbor Laboratory Press, oder Ausubel, F.M., et al. (1994) "Current Protocols in Molecular Biology", John Wiley & Sons, beschrieben.

Herstellung spezifischer Antikörper beispielsweise unter

### Beispiel 6: Northern-Hybridisierung

Für die RNA-Hybridisierung wurden 20 mg Gesamt-RNA oder 1 mg poly(A)⁺-RNA mittels Gelelektrophorese in Agarosegelen mit einer Stärke von 1,25 % unter Verwendung von Formaldehyd, wie beschrieben in Amasino (1986, Anal. Biochem. 152, 304) aufgetrennt, mittels Kapillaranziehung unter Verwendung von 10 x SSC auf positiv geladene Nylonmembranen (Hybond N+, Amersham, Braunschweig) übertragen, mittels UV-Licht immobilisiert und 3 Stunden bei 68_C unter Verwendung von Hybridisierungspuffer (10 % Dextransulfat Gew./Vol., 1 M NaCl, 1 % SDS, 100 mg Heringssperma-DNA) vorhybridisiert. Die Markierung der DNA-Sonde mit dem Highprime DNA labeling-Kit (Roche, Mannheim, Deutschland) erfolgte während der Vorhybridisierung unter Verwendung von alpha-³²P-dCTP (Amersham, Braunschweig, Deutschland). Die Hybridisierung wurde nach Zugabe der markierten DNA-Sonde im gleichen Puffer bei 68_C über Nacht durchgeführt. Die Waschschritte wurden zweimal für 15 min unter Verwendung von 2 X SSC und zweimal für 30 min unter Verwendung von 1 X SSC, 1 % SDS, bei 68_C durchgeführt. Die Exposition der verschlossenen Filter wurde bei -70_C für einen Zeitraum von 1 bis 14 T durchgeführt.

### Beispiel 7: Plasmide für die Pflanzentransformation

Zur Pflanzentransformation können binäre Vektoren, wie pBinAR verwendet werden (Höfgen und Willmitzer, Plant Science 66 (1990) 221-230). Die Konstruktion der binären Vektoren kann durch Ligation der cDNA in Sense- oder Antisense-Orientierung in T-DNA erfolgen. 5' der cDNA aktiviert ein Pflanzenpromotor die Transkription der cDNA. Eine Polyadenylierungssequenz befindet sich 3' von der cDNA.

Die gewebespezifische Expression lässt sich unter Verwendung eines gewebespezifischen Promotors erzielen. Beispielsweise kann die samenspezifische Expression erreicht werden, indem der Napin- oder der LeB4- oder der USP-Promotor 5' der cDNA einkloniert wird. Auch jedes andere samenspezifische Promotorelement kann verwendet werden. Zur konstitutiven Expression in der ganzen Pflanzen lässt sich der CaMV-35S-Promotor verwenden.

Das exprimierte Protein kann unter Verwendung eines Signalpeptids, beispielsweise für Plastiden, Mitochondrien oder das Endoplasmatische Retikulum, in ein zelluläres Kompartiment dirigiert werden (Kermode, Crit. Rev. Plant Sci. 15, 4 (1996) 285-423). Das Signalpeptid wird 5' im Leseraster mit der cDNA einkloniert, um die subzelluläre Lokalisierung des Fusionsprotein zu erreichen.

### Beispiel 8: Transformation von Agrobacterium

Die Agrobacterium-vermittelte Pflanzentransformation kann zum Beispiel unter Verwendung des GV3101- (pMP90-) (Koncz und Schell, Mol. Gen. Genet. 204 (1986) 383-396) oder LBA4404- (Clontech) Agrobacterium tumefaciens-Stamms durchgeführt werden. Die Transformation kann durch Standard-Transformationstechniken durchgeführt werden (Deblaere et al., Nucl. Acids. Tes. 13 (1984), 4777-4788).

### Beispiel 9: Pflanzentransformation

Die Agrobacterium-vermittelte Pflanzentransformation kann unter Verwendung von Standard-Transformations- und Regenerationstechniken durchgeführt werden (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, Boca Raton: CRC Press, 1993, 360 S., ISBN 0-8493-5164-2).

Beispielsweise kann Raps mittels Kotyledonen- oder Hypokotyltransformation transformiert werden (Moloney et al., Plant Cell Report 8 (1989) 238-242; De Block et al., Plant Physiol. 91 (1989) 694-701). Die Verwendung von Antibiotika für die Agrobacterium- und Pflanzenselektion hängt von dem für die Transformation verwendeten binären Vektor und Agrobacterium-Stamm ab. Die Rapsselektion wird gewöhnlich unter Verwendung von Kanamycin als selektierbarem Pflanzenmarker durchgeführt.

Der Agrobacterium-vermittelte Gentransfer in Flachs lässt sich unter Verwendung von beispielsweise einer von Mlynarova et al. (1994) Plant Cell Report 13:282-285 beschriebenen Technik durchführen.

Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

### Beispiel 10: In vivo-Mutagenese

Die in vivo-Mutagenese von Mikroorganismen kann mittels Passage der Plasmid- (oder einer anderen Vektor-) DNA durch E. coli oder andere Mikroorganismen (z.B. Bacillus spp. oder Hefen, wie Saccharomyces cerevisiae), bei denen die Fähigkeiten, die Unversehrtheit ihrer genetischen Information aufrechtzuerhalten, gestört ist, erfolgen. Übliche Mutator-Stämme haben Mutationen in den Genen für das DNA-Reparatursystem (z.B. mutHLS, mutD, mutT usw.; als Literaturstelle siehe Rupp, W.D. (1996) DNA repair mechanisms, in: Escherichia coli and Salmonella, S. 2277-2294, ASM: Washington). Diese Stämme sind dem Fachmann bekannt. Die Verwendung dieser Stämme ist beispielsweise in Greener, A., und Callahan, M. (1994) Strategies 7:32-34, erläutert. Der Transfer mutierter DNA-Moleküle in Pflanzen erfolgt vorzugsweise nach Selektion und Test der Mikrooganismen. Transgene Pflanzen werden nach verschiedenen Beispielen im Beispielteil dieses Dokumentes erzeugt.

### Beispiel 11: Untersuchung der Expression eines rekombinanten Genproduktes in einem transformierten Organismus

Die Aktivität eines rekombinanten Genproduktes im transformierten Wirtsorganismus wurde auf der Transkriptions- und/oder der Translationsebene gemessen.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots (als Bezugsstelle siehe Ausubel et al. (1988) Current Protocols in Molecular Biology, Wiley: New York, oder den oben erwähnten Beispielteil), wobei ein Primer, der so gestaltet ist, dass er an das Gen von Interesse bindet, mit einer nachweisbaren Markierung (gewöhnlich radioaktiv oder chemilumineszent) markiert wird, so dass, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E.R., et al. (1992) Mol. Microbiol. 6:317-326 beschriebene, präpariert werden.

Zur Untersuchung des Vorliegens oder der relativen Menge an von dieser mRNA translatiertem Protein können Standardtechniken, wie ein Western-Blot, eingesetzt werden (siehe beispielsweise Ausubel et al. (1988) Current Protocols in Molecular Biology, Wiley: New York). Bei diesem Verfahren werden die zellulären Gesamt-Proteine extrahiert, mittels Gelelektrophorese aufgetrennt, auf eine Matrix, wie Nitrozellulose, übertragen und mit einer Sonde, wie einem Antikörper, der spezifisch an das gewünschte Protein bindet, inkubiert. Diese Sonde ist gewöhnlich mit einer chemilumineszenten oder kolorimetrischen Markierung versehen, die sich leicht nachweisen lässt. Das Vorliegen und die Menge der beobachteten Markierung zeigt das Vorliegen und die Menge des gewünschten, in der Zelle vorliegenden mutierten Proteins an.

### Beispiel 12: Analyse der Auswirkung der rekombinanten Proteine auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353) .

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100_C erhitzt, auf Eis abgekühlt und erneut zentrifugiert, gefolgt von Extraktion in 0,5 M Schwefelsäure in Methanol mit 2 % Dimethoxypropan für 1 Std. bei 90_C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 m, 0,32 mm) bei einem Temperaturgradienten zwischen 170_C und 240_C für 20 min und 5 min bei 240_C unterworfen. Die Identität der erhaltenen Fettsäuremethylester muss unter Verwendung von Standards, die aus kommerziellen Quellen erhältlich sind (d.h. Sigma), definiert werden.

Bei Fettsäuren, für die keine Standards verfügbar sind, muss die Identität über Derivatisierung und anschließende GC-MS-Analyse gezeigt werden. Beispielsweise muss die Lokalisierung von Fettsäuren mit Dreifachbindung über GC-MS nach Derivatisierung mit 4,4-Dimethoxyoxazolin-Derivaten (Christie, 1998, siehe oben) gezeigt werden.

### Beispiel 13: Expressionskonstrukte in heterologen mikrobiellen Systemen

### Stämme, Wachstumsbedingungen und Plasmide

Der Escherichia coli-Stamm XL1 Blue MRF' kan (Stratagene) wurde zur Subklonierung der neuen Elongase piPSEl aus Phytophthora infestans verwendet. Für die funktionelle Expression dieses Gens verwendeten wir den Saccharomyces cerevisiae-Stamm INVSc 1 (Invitrogen Co.). E. coli wurde in Luria-Bertini-Brühe (LB, Duchefa, Haarlem, Niederlande) bei 37_C gezüchtet. Wenn nötig, wurde Ampicillin (100 mg/Liter) zugegeben, und 1,5 % Agar (Gew./Vol.) wurde für feste LB-Medien hinzugefügt. S. cerevisiae wurde bei 30_C entweder in YPG-Medium oder in komplettem Minimalmedium ohne Uracil (CMdum; siehe in: Ausubel, F.M., Brent, R., Kingston, R.E., Moore, D.D., Seidman, J.G., Smith, J.A., Struhl, K., Albright, L.B., Coen, D.M., und Varki, A. (1995) Current Protocols in Molecular Biology, John Wiley & Sons, New York) mit entweder 2 % (Gew./Vol.) Raffinose oder Glucose gezüchtet. Für feste Medien wurden 2 % (Gew./Vol.) Bacto™-Agar (Difco) hinzugefügt. Die zur Klonierung und Expression verwendeten Plasmide waren pUC18 (Pharmacia) und pYES2 (Invitrogen Co.).

Klonierung und Expression einer PUFA-spezifischen Elongase aus Phytophthora infestans
Für die Expression in Hefe wurde der Phytophthora infestans - cDNA-Klon piPSE1 der das PUFA-spezifische Elongase- (PSE1-) Gen kodiert, zuerst so modifiziert, dass eine KpnI-Restriktionsstelle und die Hefe-Konsensussequenz für hoch-effiziente Translation (Kozak, M. (1986) Point mutations define a sequence flanking the AUG initiator codon that modulates translation by eukaryotic ribosomes, Cell 44, 283-292) neben dem Startcodon und eine XbaI-Restriktionsstelle, die das Stopcodon flankierte, erhalten wurden. Zur Amplifikation des offenen Leserahmens wurde ein Primerpaar, das komplementär zu dessen 5'- und 3'-Enden war, synthetisiert.

| | |
|---|---|
| ppex1f: | cggggtaccacataatgtcgactgagctactgcag |
| ppex1r: | cactagtctagattccaacttcttcttcgattcc |

Die PCR-Reaktion wurde mit Plasmid-DNA als Matrize in einem Thermocycler (Biometra) mit der Pfu-DNA- (Stratagene) Polymerase und dem folgenden Temperaturprogramm durchgeführt: 3 min bei 96_C, gefolgt von 30 Zyklen mit 30 s bei 96_C, 30 s bei 55_C und 2 min bei 72_C, 1 Zyklus mit 10 min bei 72_C und Stop bei 4_C.

Die korrekte Größe des amplifizierten DNA-Fragments von 883 bp wurde mittels Agarose-TBE-Gelelektrophorese bestätigt. Die amplifizierte DNA wurde aus dem Gel mit dem QIAquick-Gelextraktionskit (QIAGEN) extrahiert und in die SmaI-Restriktionsstelle des dephosphorylierten Vektors pUC18 unter Verwendung des Sure Clone Ligation Kit (Pharmacia) ligiert, wobei pUCPSE1 erhalten wurde. Nach der Transformation von E. coli XL1 Blue MRF' kan wurde eine DNA-Minipräparation (Riggs, M.G., & McLachlan, A. (1986) A simplified screening procedure for large numbers of plasmid mini-preparation. BioTechniques 4, 310-313) an 24 ampicillinresistenten Transformanten durchgeführt, und positive Klone wurden mittels BamHI-Restriktionsanalyse identifiziert. Die Sequenz des klonierten PCR-Produktes wurde mittels Resequenzierung unter Verwendung des ABI PRISM Big Dye Terminator Cycle Sequencing Ready Reaction Kit (Perkin-Elmer, Weiterstadt) bestätigt.

Die Plasmid-DNA von pUC-PSE1 wurde zudem mit KpnI/XbaI gespalten und das erhaltene ~900 bp-Fragment in die KpnI/XbaI - Restriktionsstelle des dephosphorylierten Hefe-E. coli-Shuttlevektors pYES2 ligiert, wobei pY2PSEl erhalten wurde. Nach der Transformation von E. coli und DNA-Minipräparation aus den Transformaten wurde die Orientierung des DNA-Fragments im Vektor durch HindIII-Spaltung überprüft. Ein Klon wurde für die DNA-Maxipräparation mit dem Nucleobond^{®} AX 500 Plasmid-DNA-Extraktionskit (Macherey-Nagel, Düringen) gezüchtet.

Saccharomyces INVSc1 wurde mit pY2PSE1 und pYES2 mittels eines modifizierten PEG/Lithiumacetat-Protokolls transformiert (Ausubel et al., 1995). Nach der Selektion auf CMdum-Agarplatten mit 2 % Glucose wurden vier pY2PSE11-Transformanten (pY2PSE1a-d) und eine pYES2-Transformante zur weiteren Züchtung und funktionellen Expression ausgewählt.

Funktionelle Expression einer Elongaseaktivität in Hefe

### Vorkultur:

20 ml CMdum-Flüssigmedium mit 2 % (Gew./Vol.) Raffinose wurden mit den transgenen Hefeklonen (pY2PSE1a-d, pYES2) angeimpft und 3 T bei 30_C, 200 rpm gezüchtet, bis eine optische Dichte bei 600 nm (OD₆₀₀) von 1,5-2 erreicht war.

### Hauptkultur:

Für die Expression wurden 20 ml CMdum-Flüssigmedium mit 2 % Raffinose und 1 % (Vol./Vol.) Tergitol NP-40 mit γ-Linolsäure (γ-18:3) auf eine Endkonzentration von 0,003 % (Gew./Vol.) angereichert. Die Medien wurden mit den Vorkulturen auf eine OD₆₀0von 0,05 angeimpft. Die Expression wurde bei einer OD₆₀₀ von 0,2 mit 2 % (Gew./Vol.) Galaktose für 16 Std. induziert, wonach die Kulturen eine OD₆₀₀ von 0,8-1,2 erreicht hatten.

### Fettsäureanalyse

Die Gesamt-Fettsäuren wurden aus Hefekulturen extrahiert und mittels Gaschromatographie analysiert. Davon wurden Zellen von 5 ml Kultur mittels Zentrifugation (1000 x g, 10 min, 4_C) geerntet und einmal mit 100 mM NaHCO₃, pH 8,0, gewaschen, um restliches Medium und Fettsäuren zu entfernen. Zur Herstellung des Fettsäuremethylester (FAMES) wurden die Zellsedimente mit 1 M methanolischer H₂SO₄ und 2 % (Vol./Vol.) Dimethoxypropan für 1 Std. bei 80_C behandelt. Die FAMES wurden zweimal mit 2 ml Petrolether extrahiert, einmal mit 100 mM NaHCO₃, pH 8,0, und einmal mit destilliertem Wasser gewaschen und mit Na₂SO₄ getrocknet. Das organische Lösungsmittel wurde unter einem Argonstrom verdampft, und die FAMES wurden in 50 ∝1 Petrolether gelöst. Die Proben wurden auf einer ZEBRON-ZB-Wax-Kapillarsäule (30 m, 0,32 mm, 0,25 ∝m; Phenomenex) in einem Hewlett Packard-6850-Gaschromatograph mit einem Flammenionisationsdetektor aufgetrennt. Die Ofentemperatur wurde von 70_C (1 min halten) bis 200_C mit einer Rate von 20_C/min, dann auf 250_C (5 min halten) mit einer Rate von 5-C/min und schließlich auf 260_C mit einer Rate von 5_C/min programmiert. Stickstoff wurde als Trägergas verwendet (4,5 ml/min bei 70_C). Die Fettsäuren wurden durch Vergleich mit Retentionszeiten von FAME-Standards (SIGMA) identifiziert.

### Expressionsanalyse

Die Fettsäuremuster von fünf transgenen Hefestämmen in Mol.-% sind in Tabelle I gezeigt.

Die Verhältnisse der zugegebenen und aufgenommenen γ-Linolensäure sind durch fettgedruckte Zahlen hervorgehoben, die der elongierten Produkte durch rote Zahlen und die der elongierten γ-Linolensäure durch fettgefruckte Zahlen (letzte Zeile). Die GC-Analyse von FAMES, die aus Gesamt-Lipiden der mit pYES2 (i/Kontrolle) und pY2PSE1 transformierten Hefen ist in Figur 1 gezeigt. Für die Analyse wurden die transgenen Hefen in Anwesenheit von γ-18:3 gezüchtet..

Die Ergebnisse zeigen, dass γ-18:3 in großen Mengen in alle transgenen Hefen eingebaut worden ist. Alle vier transgenen Hefeklone, die mit pY2PSE1 transformiert wurden, zeigen einen zusätzlichen Peak im Gaschromatogramm, der durch Vergleich der Retentionszeiten als 20:3^{Δ8,11,14} identifiziert wurde. Eine Gaschromatographie/Massenspektroskopie kann zusätzliche Unterstützung zur Bestätigung dieser Identität liefern.

Die identifizierten Produkte zeigten, dass die Nukleotidsequenz von PiPSE1 eine Δ⁶-selektive Fettsäure-Elongase aus dem Moos Physcomitrella patens kodiert, die zur Bildung neuer Fettsäuren in transgenen Hefen führt.

Weitere Fütterungsexperimente mit verschiedenen anderen Fettsäuren (z.B. Linolsäure (18:2^{Δ9,12?}), Stearidonsäure (18:4^{Δ6,9,12,15})) können zur detaillierteren Bestätigung der Substratselektivität dieser Elongase durchgeführt werden.

### Beispiel 14: Reinigung des gewünschten Produktes aus transformierten Organismen allgemein

Die Gewinnung des gewünschten Produktes aus Pflanzenmaterial oder Pilzen, Algen, Ciliaten, tierischen Zellen oder aus dem Überstand der vorstehend beschriebenen Kulturen kann durch verschiedene, im Fachgebiet bekannte Verfahren erfolgen. Wird das gewünschte Produkt nicht aus den Zellen sezerniert, können die Zellen aus der Kultur durch langsame Zentrifugation geerntet werden, die Zellen können durch Standardtechniken, wie mechanische Kraft oder Ultraschallbehandlung, lysiert werden. Organe von Pflanzen können mechanisch von anderem Gewebe oder anderen Organen getrennt werden. Nach der Homogenisation werden die Zelltrümmer durch Zentrifugation entfernt, und die Überstandsfraktion, welche die löslichen Proteine enthält, wird zur weiteren Reinigung der gewünschten Verbindung aufbewahrt. Wird das Produkt aus gewünschten Zellen sezerniert, werden die Zellen durch langsame Zentrifugation aus der Kultur entfernt, und die Überstandsfraktion wird zur weiteren Reinigung aufbewahrt.

Die Überstandsfraktion aus jedem Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder die Verunreinigungen auf dem Harz zurückbleiben, die Probe hingegen nicht. Diese Chromatographieschritte können wenn nötig wiederholt werden, wobei die gleichen oder andere Chromatographieharze verwendet werden. Der Fachmann ist in der Auswahl geeigneter Chromatographieharze und ihrer wirksamsten Anwendung für ein bestimmtes zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet ist ein breites Spektrum an Reinigungsverfahren bekannt, und das vorstehende Reinigungsverfahren soll nicht beschränkend sein. Diese Reinigungsverfahren sind zum Beispiel beschrieben in Bailey, J.E., & Ollis, D.F., Biochemical Engineering Fundamentals, McGraw-Hill: New York (1986).

Die Identität und Reinheit der isolierten Verbindungen kann durch Standardtechniken des Fachgebiets bestimmt werden. Dazu gehören Hochleistungs-Flüssigkeitschromatographie (HPLC), spektroskopische Verfahren, Färbeverfahren, Dünnschichtchromatographie, NIRS, Enzymtest oder mikrobiologisch. Eine Übersicht über diese Analyseverfahren siehe in: Patek et al. (1994) Appl. Environ. Microbiol. 60:133-140; Malakhova et al. (1996) Biotekhnologiya 11:27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A., et al. (1987) Applications of HPLC in Biochemistry in: Laboratory Techniques in Biochemistry and Molecular Biology, Bd. 17.

## Patentansprüche

1. Verfahren zur Herstellung von C₂₀ oder C₂₂ Fettsäuremolekülen mit mindestens zwei Doppelbindungen im Fettsäuremolekül, wobei das Verfahren das Züchten eines Mirkoorganismusses oder einer Pflanze umfasst, der/die eine Nukleinsäure umfasst, die ein Polypeptid kodiert, das C₁₆ oder C₁₈ Fettsäuren mit mindestens zwei Doppelbindungen in der Fettsäure um mindestens zwei Kohlenstoffatome verlängert, wobei die Fettsäuren C_{18:3}^{Δ6,9,12}, C_{18:4}^{Δ6,9,12,15} und C_{16;3}^{Δ7,10,13} mindestens um den Faktor 1,5 bevorzugt werden gegenüber den ungesättigten Fettsäuren C_{18:2}^{Δ9,12}, C_{18:3}^{Δ4,7,10}, C_{18:3}^{Δ5,8,11}, C_{18:3}^{Δ7,10,13}, C_{18:3}^{Δ8,11,14}, C_{18:3}^{Δ9,12,15} oder C_{18:3}^{Δ5,c9,12}, wobei die Fettsäuren C_{18:3}^{Δ5t,9,12}, C20:3^{Δ8,11,14}, C_{20:4}^{Δ5,8,11,14} und C_{20:5}^{Δ5,8,11,14,17} nicht verlängert werden und wobei die Nukleinsäure ausgewählt ist aus der Gruppe, bestehend aus
a) einer in SEQ ID NO:1 dargestellten Nukleinsäuresequenz,
b) einer Nukleinsäuresequenz, die sich gemäß dem degenerierten genetischen Code von der in SEQ ID NO:2 dargestellten Sequenz ableitet,
c) Derivate der in SEQ ID NO:1 dargestellten Sequenz, die Polypeptide kodieren die zu mindestens 50% zu der in SEQ ID NO:2 dargestellten Aminosäuresequenz identisch sind.

2. Verfahren nach Anspruch 1, wobei die C₂₀- oder C₂₂-Fettsäuremoleküle aus dem Organismus in Form eines Öls, Lipids oder einer freien Fettsäure isoliert werden.

3. Verwendung einer Nukleinsäure wie in Anspruch 1 beschrieben zur Verlängerung von C₁₆- oder C₁₈-Fettsäuren mit mindestens zwei Doppelbindungen in der Fettsäure um mindestens zwei Kohlenstoffatome, wobei die Fettsäuren C_{18:3}^{Δ6,9,12}, C_{18:4}^{Δ6,9,12,15} und C_{16:3}^{Δ7,10,13} mindestens um den Faktor 1,5 bevorzugt werden gegenüber den ungesättigten Fettsäuren C_{18:2}^{Δ9,12}, C_{18:3}^{Δ4,7,10}, C_{18:3}^{Δ5,8,11}, C_{18:3}^{Δ7,10,13}, C_{18:3}^{Δ8,11,14}, C_{18:3}^{Δ9,12,15} oder C_{18:3}^{Δ5,c9,12} und wobei die Fettsäuren C₁₈:₃^{Δ5t,9,12}, C_{20:3}^{Δ8,11,14}, C_{20:4}^{Δ5,8,11,14} und C_{20:5}^{Δ5,8,11,17} nicht verlängert werden.

## Claims

1. A process for the production of C₂₀ or C₂₂ fatty acid molecules with at least two double bonds in the fatty acid molecule, which comprises culturing a microorganism or a plant which comprises a nucleic acid encoding a polypeptide which elongates C₁₆ or C₁₈ fatty acids with at least two double bonds in the fatty acid by at least two carbon atoms, there being a preference for the fatty acids C_{18:3}^{Δ6,9,12}, C_{18:4}^{Δ6,9,12,15} and C_{16:3}^{Δ7,10,13} which exceeds that for the unsaturated fatty acids C_{18:2}^{Δ9,12}, C_{18:3}^{Δ4,7,10}, C_{18:3}^{Δ5,8,11}, C_{18:3}^{Δ7,10,13}, C_{18:3}^{Δ8,11,14}, C_{18:3}^{Δ9,12,15} or C_{18:3}^{Δ5,c9,12} by at least a factor of 1.5, the fatty acids C_{18:3}^{Δ5t,9,12} C_{20:3}^{Δ8,11,14}, C_{20:4}^{Δ5,8,11,14} and C_{20:5}^{Δ5,8,11,14,17} not bering elongated and the nucleic acid being selected from the group consisting of
a) a nucleic acid sequence shown in SEQ ID NO:1,
b) a nucleic acid sequence which, in accordance with the degeneracy of the genetic code, is derived from the sequence shown in SEQ ID N0:2,
c) derivatives of the sequence shown in SEQ ID NO:1 which encode polypeptides which are at least 50% identical to the amino acid sequence shown in SEQ ID NO:2.

2. The process according to claim 1, wherein the C₂₀ or C₂₂ fatty acid molecules are isolated from the organism in the form of an oil, lipid or a free fatty acid.

3. The use of a nucleic acid as described in claim 1 for elongating C₁₆ or C₁₈ fatty acids with at least two double bonds in the fatty acid by at least two carbon atoms, there being a preference for the fatty acids C_{18:3}^{Δ6,9,12}, C_{18:4}^{Δ6,9,12,15} and C_{16:3}^{Δ7,10,13} which exceeds that for the unsaturated fatty acids C_{18:2}^{Δ9,12}, C_{18:3}^{Δ4,7,10}, C_{18:3}^{Δ5,8,11}, C_{18:3}^{Δ7,10,13}, C_{18:3}^{Δ8,11,14}, C_{18:3}^{Δ9,12,15} or C_{18:3}^{Δ5,c9,12} by at least a factor of 1.5, and the fatty acids C_{18:3}^{Δ5t,9,12}, C_{20:3}^{Δ8,11,14}, C_{20:4}^{Δ5,8,11,14} and C_{20:5}^{Δ5,8,11,14,17} not being elongated.

## Revendications

1. Procédé pour la préparation de molécules d'acides gras en C₂₀ ou C₂₂ comportant au moins deux doubles liaisons dans la molécule d'acide gras, le procédé comprenant la culture d'un micro-organisme ou d'une plante qui comprennent un acide nucléique codant pour un polypeptide qui prolonge d'au moins deux atomes de carbone des acides gras en C₁₆ ou C₁₈ comportant au moins deux doubles liaisons dans l' acide gras, les acides gras C_{18:3}^{Δ6,9,12}, C_{18.4}^{Δ6,9,12,15} et C_{16:3}^{Δ7,10,13} étant préférés par au moins le facteur 1,5 par rapport aux acides gras insaturés C_{18:2} ^{Δ9,12}, C_{18:3}^{Δ4,7,10} C_{18:3}^{Δ5,8,11}, C_{18:3}^{Δ7,10,13}, C_{18:3}^{Δ8,11,14}, C_{18:3}^{Δ,9,12,15} ou C_{18:3}^{Δ5,c9,12}, les acides gras C_{18:3}^{Δ5t,9,12} C_{20:3}^{Δ8,11,14}, C_{20:4}^{Δ5,8,11,14} et C_{20:5}^{Δ5,8,11,14,17} n'étant pas prolongés et l'acide nucléique étant choisi dans le groupe constitué par
a) une séquence d'acide nucléique représentée dans SEQ ID n° 1,
b) une séquence d'acide nucléique qui dérive, selon le code génétique dégénéré, de la séquence représentée dans SEQ ID n° 2,
c) des dérivés de la séquence représentée dans SEQ ID n° 1, qui codent pour des polypeptides qui sont identiques à raison d'au moins 50 % à la séquence d'acide nucléique représentée dans SEQ ID n° 2.

2. Procédé selon la revendication 1, dans lequel les molécules d'acides gras en C₂₀ ou C₂₂ sont isolées, à partir de l'organisme, sous forme d'une huile, d'un lipide ou d'un acide gras libre.

3. Utilisation d'un acide nucléique tel que décrit dans la revendication 1, pour le prolongement par au moins deux atomes de carbone d'acides gras en C₁₆ ou C₁₈ comportant au moins deux doubles liaisons dans l'acide gras, les acides gras C_{18:3}^{Δ6,9,12}, C_{18:4}^{Δ6,6,9,12,15} et C_{16:3}^{Δ7,10,13} étant préférés par au moins le facteur 1,5 par rapport aux acides gras insaturés C_{18:2}^{Δ9,12}, _{C18}^{Δ4,7,10} C_{18:3}^{Δ5,8,11}, C_{18:3}^{Δ7,10,13}, C_{18:3}^{Δ8,11,14}, C_{18:3}^{Δ,9,12,15} ou C_{18:3}^{Δ5,c9,12} et les acides gras C_{18:3}^{Δ5t,9,12,} C_{20:3}^{Δ8,11,14}, C_{20:4}^{Δ5,8,11,14} et C_{20:5}^{Δ5,8,11,14,17} n'étant pas prolongés.


### SEQUENZPROTOKOLL

<110> BASF Plant Science GmbH
<120> Neues Elongasegen und verfahren zur Herstellung mehrfach unges#ttigter Fetts#uren
<130> 925_2001
<140>
   <141>
<160> 2
<170> PatentIn Vers. 2.0
<210> 1
   <211> 1066
   <212> DNA
   <213> Phytophthora infestans
<220>
   <221> CDS
   <222> (52)..(888)
<400> 1
   gaattcggca cgaggttcgc acgtccatcg tctactcacc aacaagaagt c atg tcg   57
                                                            Met Ser
                                                              1
   act gag cta ctg cag agc tac tac gcg tgg gcc aac gcc acg gag gcc    105
   Thr Glu Leu Leu Gln Ser Tyr Tyr Ala Trp Ala Asn Ala Thr Glu Ala
           5                   10                  15
   aag ctg ctg gac tgg gtc gac cct gag ggc ggc tgg aag gtg cat cct    153
   Lys Leu Leu Asp Trp Val Asp Pro Glu Gly Gly Trp Lys Val His Pro
       20                  25                  30
   atg gca gac tac ccc cta gcc aac ttc tcc agc gtc tac gcc atc tgc    201
   Met Ala Asp Tyr Pro Leu Ala Asn Phe Ser Ser Val Tyr Ala Ile Cys
   35                  40                  45                  50
   gtc gga tac ttg ctc ttc gta atc ttc ggc acg gcc ctg atg aaa atg    249
   Val Gly Tyr Leu Leu Phe Val Ile Phe Gly Thr Ala Leu Met Lys Met
                   55                  60                  65
   gga gtc ccc gcc atc aag acc agt cca tta cag ttt gtg tac aac ccc    297
   Gly Val Pro Ala Ile Lys Thr Ser Pro Leu Gln Phe Val Tyr Asn Pro
               70                  75                  80
   atc caa gtc att gcc tgc tct tat atg tgc gtg gag gcc gcc atc cag    345
   Ile Gln Val Ile Ala Cys Ser Tyr Met Cys Val Glu Ala Ala Ile Gln
           85                  90                  95
   gcc tac cgc aac ggc tac acc gcc gcc ccg tgc aac gcc ttt aag tcc    393
   Ala Tyr Arg Asn Gly Tyr Thr Ala Ala Pro Cys Asn Ala Phe Lys Ser
       100                 105                 110
   gac gac ccc gtc atg ggc aac gtt ctg tac ctc ttc tat ctc tcc aag    441
   Asp Asp Pro Val Met Gly Asn Val Leu Tyr Leu Phe Tyr Leu Ser Lys
   115                 120                 125                 130
   atg ctc gac ctg tgc gac aca gtc ttc att atc cta gga aag aag tgg    489
   Met Leu Asp Leu Cys Asp Thr Val Phe Ile Ile Leu Gly Lys Lys Trp
                   135                 140                 145
   aaa cag ctt tcc atc ttg cac gtg tac cac cac ctt acc gtg ctt ttc    537
   Lys Gln Leu Ser Ile Leu His Val Tyr His His Leu Thr Val Leu Phe
                       150                      155                      160
   gtc tac tat gtg acg ttc cgc gcc gct cag gac ggg gac tca tat gct     585
   Val Tyr Tyr Val Thr Phe Arg Ala Ala Gln Asp Gly Asp Ser Tyr Ala
           165                 170                 175
   acc atc gtg ctc aac ggc ttc gtg cac acc atc atg tac act tac tac     633
   Thr Ile Val Leu Asn Gly Phe Val His Thr Ile Met Tyr Thr Tyr Tyr
       180                 185                 190
   ttc gtc agc gcc cac acg cgc aac att tgg tgg aag aag tac ctc acg     681
   Phe Val Ser Ala His Thr Arg Asn Ile Trp Trp Lys Lys Tyr Leu Thr
   195                 200                 205                 210
   cgc att cag ctt atc cag ttc gtg acc atg aac gtg cag ggc tac ctg     729
   Arg Ile Gln Leu Ile Gln Phe Val Thr Met Asn Val Gln Gly Tyr Leu
                   215                 220                 225
   acc tac tct cga cag tgc cca ggc atg cct cct aag gtg ccg ctc atg     777
   Thr Tyr Ser Arg Gln Cys Pro Gly Met Pro Pro Lys Val Pro Leu Met
               230                 235                 240
   tac ctt gtg tac gtg cag tca ctc ttc tgg ctc ttc atg aat ttc tac     825
   Tyr Leu Val Tyr Val Gln Ser Leu Phe Trp Leu Phe Met Asn Phe Tyr
           245                 250                 255
   att cgc gcg tac gtg ttc ggc ccc aag aaa ccg gcc gtg gag gaa tcg     873
   Ile Arg Ala Tyr Val Phe Gly Pro Lys Lys Pro Ala Val Glu Glu Ser
       260                 265                 270
   aag aag aag ttg taa cggcgcttgt taaaaagtct aacctcgctg taacagctta    928
   Lys Lys Lys Leu
   275
   aaacacacac acacacaacg ctttgtagag gaggtaagta gtggcaactc gtgtagaaat 988
   gcagcatgcc catcaaatac atcccgtatg attcaaaaaa aaaaaaaaaa aaaaaaaaaa 1048
   aaaaaaaaaa aactcgag                                               1066
<210> 2
   <211> 278
   <212> PRT
   <213> Phytophthora infestans
<400> 2
   Met Ser Thr Glu Leu Leu Gln Ser Tyr Tyr Ala Trp Ala Asn Ala Thr
   1 5 10 15
   Glu Ala Lys Leu Leu Asp Trp Val Asp Pro Glu Gly Gly Trp Lys Val
   20 25 30
   His Pro Met Ala Asp Tyr Pro Leu Ala Asn Phe Ser Ser Val Tyr Ala
   35 40 45
   Ile Cys Val Gly Tyr Leu Leu Phe Val Ile Phe Gly Thr Ala Leu Met
   50 55 60
   Lys Met Gly Val Pro Ala Ile Lys Thr Ser Pro Leu Gln Phe Val Tyr
   65 70 75 80
   Asn Pro Ile Gln Val Ile Ala Cys Ser Tyr Met Cys Val Glu Ala Ala
   85 90 95
   Ile Gln Ala Tyr Arg Asn Gly Tyr Thr Ala Ala Pro Cys Asn Ala Phe
   100 105 110
   Lys Ser Asp Asp Pro Val Met Gly Asn Val Leu Tyr Leu Phe Tyr Leu
   115 120 125
   Ser Lys Met Leu Asp Leu Cys Asp Thr Val Phe Ile Ile Leu Gly Lys
   130 135 140
   Lys Trp Lys Gln Leu Ser Ile Leu His Val Tyr His His Leu Thr Val
   145 150 155 160
   Leu Phe Val Tyr Tyr Val Thr Phe Arg Ala Ala Gln Asp Gly Asp Ser
   165 170 175
   Tyr Ala Thr Ile Val Leu Asn Gly Phe Val His Thr Ile Met Tyr Thr
   180 185 190
   Tyr Tyr Phe Val Ser Ala His Thr Arg Asn Ile Trp Trp Lys Lys Tyr
   195 200 205
   Leu Thr Arg Ile Gln Leu Ile Gln Phe Val Thr Met Asn Val Gln Gly
   210 215 220
   Tyr Leu Thr Tyr Ser Arg Gln Cys Pro Gly Met Pro Pro Lys Val Pro
   225 230 235 240
   Leu Met Tyr Leu Val Tyr Val Gln Ser Leu Phe Trp Leu Phe Met Asn
   245 250 255
   Phe Tyr Ile Arg Ala Tyr Val Phe Gly Pro Lys Lys Pro Ala Val Glu
   260 265 270
   Glu Ser Lys Lys Lys Leu
   275